(19) Europäisches Patentamt
European Patent Office
Office européen des brevets

(11) Veröffentlichungsnummer: **0 299 397 A2**

# (12) EUROPÄISCHE PATENTANMELDUNG

(21) Anmeldenummer: **88111011.8**

(51) Int. Cl.[4]: **C07K 7/10 , A61K 37/02**

(22) Anmeldetag: **09.07.88**

Patentansprüche für folgende Vertragsstaaten: ES + GR.

(30) Priorität: **16.07.87 DE 3723551**

(43) Veröffentlichungstag der Anmeldung: **18.01.89 Patentblatt 89/03**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Breipohl, Gerhard, Dr.**
**Geisenheimer Strasse 95**
**D-6000 Frakfurt am Main 71(DE)**
Erfinder: **Knolle, Jochen, Dr.**
**Höchster Strasse 21**
**D-6239 Kriftel(DE)**
Erfinder: **König, Wolfgang, Dr.**
**Eppsteiner Strasse 25**
**D-6238 Hofheim am Taunus(DE)**
Erfinder: **Hropot, Max, Dr.**
**Friedrich-Stolz-Strasse 13**
**D-6093 Flörsheim am Main(DE)**

(54) **Peptide mit vasorelaxierender, natriuretischer und diuretischer Wirkung, Verfahren zu ihrer Herstellung, diese enthaltende Mittel und ihre Verwendung.**

(57) Die Erfindung betrifft neue Peptide der Formel

$$X-V-A-A^1-A^2-B-C-D-B-C-Gly-Ala-E-F-G-Leu-Gly-Cys-Z$$

in der X, A, A¹, A², B, C, D, E, F, G und Z in der Beschreibung angegebene Bedeutung haben, Verfahren zu ihrer Herstellung, diese enthaltende Mittel und ihre Verwendung.

EP 0 299 397 A2

# Peptide mit vasorelaxierender, natriuretischer und diuretischer Wirkung, Verfahren zu ihrer Herstellung, diese enthaltende Mittel und ihre Verwendung

Aus der EP-A2-140731, der EP-A2-142487 und der EP-A2-152333 sind Peptide bekannt, deren Sequenz eine Teilsequenz des natürlichen atrionatriuretischen Faktors (ANF) des Menschen oder der Ratte darstellt. Ferner werden ANF-Peptide in EP-A 231 752 (ZA 87/0 272) beschrieben.

Die Erfindung betrifft neue Peptide der Formel I,

$$X\text{-}\overset{3}{V}\text{-}A\text{-}A^1\text{-}A^2\text{-}B\text{-}C\text{-}D\text{-}B\text{-}C\text{-}Gly\text{-}Ala\text{-}E\text{-}F\text{-}G\text{-}Leu\text{-}Gly\text{-}\overset{19}{Cys}\text{-}Z \quad (I)$$

in welcher

X Wasserstoff, $(C_1\text{-}C_5)$-Alkoxycarbonyl, $(C_6\text{-}C_{12})$-Aryloxycarbonyl, $(C_7\text{-}C_{13})$-Aralkyloxycarbonyl, $(C_1\text{-}C_6)$-Alkanoyl, $(C_7\text{-}C_{13})$-Aroyl, Arginyl, Lysyl, $\epsilon$-Aminocaproyl, Arginyl-Arginyl, Arginyl-Lysyl, Lysyl-Arginyl oder Lysyl-Lysyl bedeutet,

gegebenenfalls in $\alpha$-Stellung verzweigtes und gegebenenfalls in $\omega$-Stellung durch Amino oder Guanidino monosubstituiertes $(C_1\text{-}C_{12})$-Alkylcarbonyl oder $(C_3\text{-}C_8)$-Cycloalkylcarbonyl bedeutet oder für Ser, Thr, Ser-(Y), Thr(Y), Q oder Leu, jeweils in ihrer L-bzw. D-Konfiguration oder Ser-Ser, Thr-Thr, Ser-Thr, Q-Ser, Q-Thr, Thr-Q, Ser-Q, Ser(Y)-Ser oder Ser-Ser(Y) steht, wobei jede Aminosäure in ihrer L-bzw. D-Konfiguration vorliegen kann und die N-terminale Aminogruppe der Aminosäure oder des Dipeptid-Restes frei vorliegt (N-terminaler Rest = H) oder durch $(C_1\text{-}C_5)$-Alkoxycarbonyl, $(C_6\text{-}C_{12})$-Aryloxycarbonyl, $(C_7\text{-}C_{13})$-Aralyloxycarbonyl, $(C_1\text{-}C_6$-Alkanoyl, $(C_7\text{-}C_{13})$-Aroyl, Arginyl, Lysyl, $\epsilon$-Aminocaproyl, Arginyl-Arginyl, Arginyl-Lysyl, Lysyl-Arginyl oder Lysyl-Lysyl acyliert ist oder X fehlen kann;

V Cys oder, falls X fehlt, $\omega$-Thio$(C_2\text{-}C_8)$alkylcarbonyl, wie beispielsweise Mercaptopropionsäure, Mercaptoessigsäure, Mercaptobuttersäure, bedeutet;

A Phe, Tyr(Me), Tyr($Bu^t$), 4-Halogenphenylalanin, Trp oder einen L-2-Thienylalanin-Rest bedeutet;

$A^1$ Gly, Ala oder D-Ala bedeutet;

$A^2$ Gly, Ala oder D-Ala bedeutet; oder

$A^1$ und $A^2$ zusammen eine gegebenenfalls verzweigte $\omega$-Amino$(C_3\text{-}C_8)$-alkylcarbonsäure bedeutet;

B Arg, Lys, Orn oder einen L-Homoarginin-Rest bedeutet;

C Ile, Met, Phe, Trp, Leu, Ser, Thr, Val, His, Pro, Asn, Ser($Bu^t$), Cyclohexylalanin, tert. Butylglycin, Neopentylglycin oder einen L-2-Thienylalanin-Rest bedeutet;

D Asp, Glu, Gln, Asn, Phe, Leu, Ile, Trp, Pro, Tyr, Ala, Asp ($OBu^t$), Asp (OBzl), Glu($OBu^t$), Glu(OBzl), einen 2-Thienylalanin-Rest, Aad, Tyr($Bu^t$) oder Tyr(Me) bedeutet, wobei die Aminosäuren jeweils in ihrer L- oder D-Konfiguration vorliegen können;

E Gln, Thr oder Pro bedeutet;

F Ser, Thr, Pro, Ala, Ser($Bu^t$) oder Thr($Bu^t$), jeweils in ihrer L- oder D-Konfiguration bedeutet;

G Gly, Ala oder D-Ala bedeutet;

Q einen Rest der Formel IV,

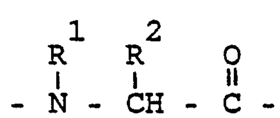 (IV)

worin

$R^1$ und $R^2$ zusammen mit den diese Reste tragenden Atomen ein heterocyclisches mono-, bi- oder tricyclisches Ringsystem mit 3 bis 15 C-Atomen bilden,

bedeutet;

Y tert.-Butyl oder einen gegebenenfalls geschützten oder teilgeschützten Glycosylrest bedeutet;

und

Z für einen Rest der Formel II steht,

-H-I-J-K-L-M (II)

worin H Asn, Ser, Q oder Thr, jeweils in ihrer L-oder D-Form, oder Gly bedeutet,

I Ser, Thr, Ala, Ser($Bu^t$), Thr($Bu^t$) oder Pro, jeweils in ihrer L- oder D-Form bedeuten;
J Phe, Trp, D-Phe, D-Trp, Tyr(Me), Cyclohexylalanyl, oder einen 2-Thienylalanin-Rest bedeutet;
K Arg, Lys, Orn oder eine Bindung bedeutet;
L Q, Gly, Tyr, Tyr($Bu^t$), Tyr(Me) oder eine Bindung bedeutet;
M Arg-OH, Arg-$NH_2$, OH, OR, $NH_2$, NHR′, Gly-Lys-Arg-OH, Gly-Lys-Arg-$NH_2$ oder L-Argininol bedeutet;
Q wie oben definiert ist;
R unverzweigtes ($C_1$-$C_6$)-Alkyl bedeutet und
R′ -$[CH_2]_n$-$NH_2$ oder -$[CH_2]_n$-NH-C(NH)$NH_2$,
wobei n ganzzahlig ist und für 3-8 steht, bedeutet; sowie deren physiologisch verträgliche Salze, wobei die der Sequenz des natürlichen ANF entsprechenden Peptide der Formel III

(III) X-Cys-Phe-Gly-Gly-Arg-C-Asp-Arg-Ile-Gly-Ala-Gln-

-Ser-Gly-Leu-Gly-Cys-Z

worin X Ser, Ser-Ser, Arg-Ser-Ser oder Arg-Arg-Ser-Ser
C Ile oder Met,
Z Asn-Ser-Phe-K-L-M,
K Arg oder eine Bindung,
L Tyr oder eine Bindung und
M OH oder $NH_2$ bedeuten sowie deren Salze,
die in EP-A 231 752 (ZA 87/0 272; HOE 86/F 099J) beschriebenen Verbindungen;
rat-[$Mac^{105}$]ANF(101-126), rat-[$Mpr^{105}$]ANF(101-126), rat-[$Mbu^{105}$]ANF (101-126) und rat-[$Mpr^{105}$, D-$Ala^{107}$] ANF(105-126))
ausgenommen sind.

Als Rest eines heterocyclischen Ringsystems der Formel IV kommen insbesondere Reste von Heterocyclen aus der folgenden Gruppe in Betracht:
Pyrrolidin (A); Piperidin (B); Tetrahydroisochinolin (C); Decahydroisochinolin (D); Octahydroindol (E); Octahydrocyclopenta[b]pyrrol (F); 2-Aza-bicyclo[2.2.2]octan (G); 2-Azabicyclo[2.2.1]heptan (H); 2-Azaspiro-[4.5]decan (I); 2-Azaspiro[4.4]nonan (J); Spiro[(bicyclo[2.2.1]heptan)-2,3-pyrrolidin] (K); Spiro[(bicyclo[2.2.2]-octan)-2,3-pyrrolidin] (L); 2-Azatricyclo[4.3.0.$1^{6,9}$]decan (M); Decahydrocyclohepta[b]pyrrol (N); Octahydroisoindol (O); Octahydrocyclopenta[c]pyrrol (P); 2,3,3a,4,5,7a-Hexahydroindol (Q); Tetrahydrothiazol (R); 2-Azabicyclo[3.1.0]hexan (S); die alle gegebenenfalls substituiert sein können.

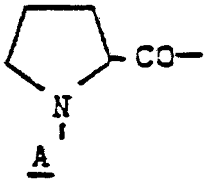

A

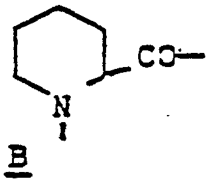

B

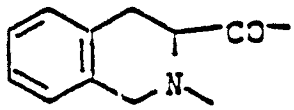

C

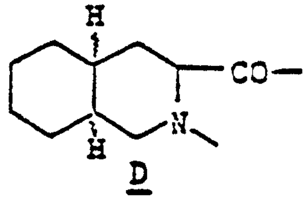

D

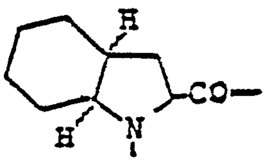

E

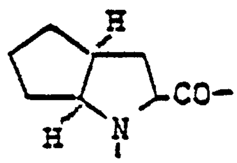

F

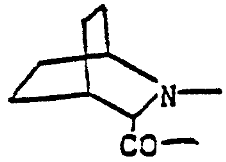

G

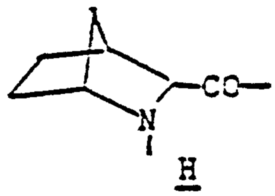

H

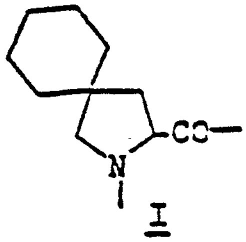

I

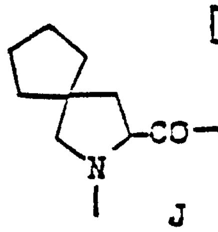

J

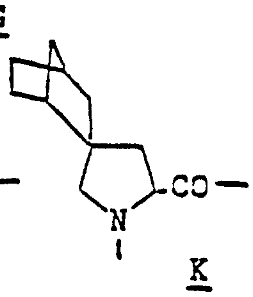

K

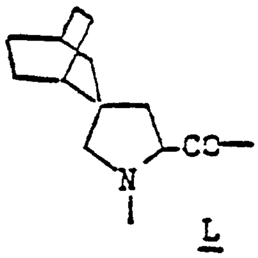

L

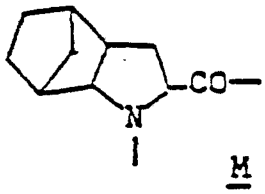

M

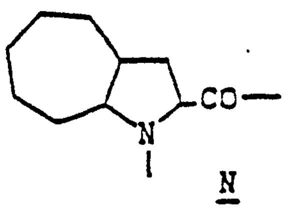

N

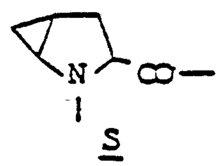

S

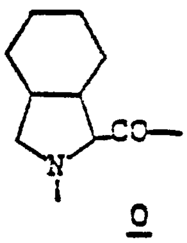

O

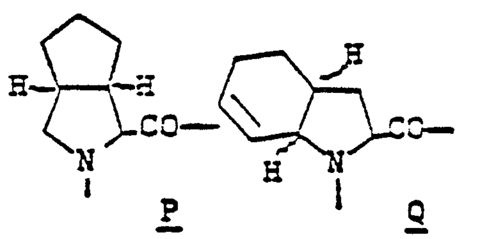

P

Q

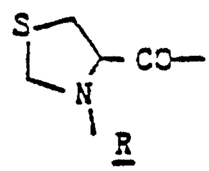

R

Die oben genannten Resten zugrundeliegenden Heterocyclen sind beispielsweise bekannt aus US-PS 4 344 949, US-PS 4 374 847, US-PS 4 350 704, EP-A 50 800, EP-A 31 741, EP-A 51 020, EP-A 49 658, EP-A 49 605, EP-A 29 488, EP-A 46 953, EP-A 52 870, EP-A 72 022, EP-A 84 164, EP-A 89 637, EP-A 90 341, EP-A 90 362, EP-A 105 102, EP-A 109 020, EP-A 111 873 und EP-A 113 880.

Y ist tert.-Butyl oder ein mit in der Kohlenhydratchemie üblichen Schutzgruppen geschützter, teilgeschützter oder ungeschützter Glycosylrest, der sich von einer Glycopyranose, Glycofuranose oder einem Oligosaccharid ableitet.

Bevorzugt sind geschützte Glycosylreste. Die Glycosylreste können sowohl α- als auch β-glycosidisch mit dem Serin-Rest verknüpft sein.

Y kann beispielsweise ein Glycofuranosyl- oder Glycopyranosyl-Rest sein, der sich von natürlich vorkommenden Aldotetrosen, Aldopentosen, Aldohexosen, Ketopentosen, Desoxyaldosen, Aminoaldosen und Oligosacchariden, wie Di-und Trisacchariden, sowie deren Stereoisomeren ableitet.

Die Glycosylreste Y leiten sich insbesondere von natürlichen, im Mikroorganismen, Pflanzen, Tieren oder Menschen vorkommenden D- oder L-Monosacchariden wie Ribose (Rib), Arabinose (Ara), Xylose (Xyl), Lyxose (Lyx), Allose (All), Altrose (Alt), Glucose (Glc), Mannose (Man), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc),

Rohodosamin (RN), N-Acetyl-glucosamin (GlcNAc), N-Acetyl-galactosamin (GalNAc), N-Acetyl-mannosamin (ManNAc) oder Disacchariden, wie Maltose (Mal), Lactose (Lac), Cellobiose (Cel), Gentiobiose (Gen), N-Acetyl-lactosamin (LacNAc), Chitobiose (Chit), β-Galactopyranosyl-(1→3)-N-acetylgalactosamin und β-Galactopyranosyl-(1→3)-oder -(1→4)-N-acetyl-glucosamin, sowie deren synthetischen Derivate, wie 2-Desoxy-, 2-Amino- 2-Acetamido- oder 2-Halogeno-, bevorzugt Bromo- und Jodo-Zucker ab.

Unter den in der Kohlenhydratchemie üblichen Schutzgruppen versteht man z.B. die $(C_1-C_{10})$-Acylschutzgruppen wie $(C_1-C_6)$-Alkanoyl (z.B. Acetyl-, Trichloroacetyl-, Trifluoracetyl-), Benzoyl- oder p-Nitrobenzoyl-, sowie gegebenenfalls modifizierte Methyl-, Methyloxymethyl-, Benzyl-, Tetrahydropyranyl-, Benzyliden-, Isopropyliden- oder Trityl-Gruppe, wobei hier die Acylschutzgruppen, insbesondere die Acetyl-(Ac-)Gruppe, bevorzugt sind.

Solche Peptide der Formel I sind bevorzugt, worin

X Wassertoff, $(C_1-C_5)$-Alkoxycarbonyl, $(C_6-C_{12})$-Aryloxycarbonyl, $(C_7-C_{13})$-Aralkyloxycarbonyl, $(C_1-C_6)$-Alkanoyl, $(C_7-C_{13})$-Aroyl, Arginyl, Lysyl oder Arginyl-Arginyl bedeutet,
gegebenenfalls in α-Stellung verzweigtes und gegebenenfalls in ω-Stellung durch Amino oder Guanidino monosubstituiertes $(C_1-C_{12})$-Alkylcarbonyl oder $(C_3-C_8)$-Cycloalkylcarbonyl bedeutet oder für Ser, Thr oder Q, jeweils in ihrer L- bzw. D-Konfirguration oder Ser-Ser, Thr-Thr, Ser-Thr, Pro-Ser, Pro-Thr, Thr-Pro oder Ser-Pro, vorzugsweise Ser-Ser steht, wobei jede Aminosäure in ihrer L- bzw. D-Konfiguration vorliegen kann und die N-terminale Aminogruppe der Aminosäure oder des Dipeptid-Restesd frei vorliegt oder durch $(C_1-C_5)$-Alkoxycarbonyl, $(C_6-C_{12})$-Aryloxycarbonyl, $(C_7-C_{13})$-Aralkyloxycarbonyl, $(C_1-C_6)$-Alkanoyl, $(C_7-C_{13})$-Aroyl, Arginyl, Lysyl oder Arginyl-Arginyl acyliert ist oder X fehlen kann;
V Cys oder, falls X fehlt, Mercaptopropionsäure, Mercaptoessigsäure oder Mercaptobuttersäure bedeutet;
A Phe, Tyr(Me), Tyr($Bu^t$), 4-Halogenphenylalanin oder einen L-2-Thienylalanin-Rest, vorzugsweise Phe bedeutet;
$A^1$ Gly, Ala oder D-Ala bedeutet;
$A^2$ Gly, Ala oder D-Ala bedeutet; oder
$A^1$ und $A^2$ zusammen eine gegebenenfalls verzweigte ω-Amino-$(C_3-C_8)$-alkylcarbonsäure bedeutet;
B Arg oder Lys bedeutet;
C Ile, Cyclohexylalanin, tert.-Butylglycin, Neopentylglycin, Phe, Leu, Val oder einen L-2-Thienylalanin-Rest bedeutet;
D Asp, Glu, Gln, Asn, Leu, Ile, Trp, Asp($OBu^t$), Glu($OBu^t$), Glu(OBzl) oder einen 2-Thienylalanin-Rest, vorzugsweise Asp, Glu oder Leu bedeutet, wobei die Aminosäuren jeweils in ihrer L- oder D-Konfiguration vorliegen können;
E Gln, Thr oder Pro bedeutet;
F Ser, Thr oder Ala, vorzugsweise Ser oder Ala, jeweils in ihrer L- oder D-Konfiguration bedeutet;
G Gly, Ala oder D-Ala bedeutet und
Z für ein Rest der Formel II steht,
worin H Asn, Ser, Pro oder Thr, vorzugsweise Pro, Thr oder Asn, jeweils in ihrer L- oder D-Form, oder Gly bedeutet;
I Ser, Thr, Ala oder Ser($Bu^t$), vorzugsweise Ser, jeweils in ihrer L- oder D-Form bedeutet;
J Phe, Tyr(Me), Cyclohexylalanyl oder einen L-2-Thienylalanin-Rest, vorzugsweise Phe bedeutet;
K Arg, Lys, Orn oder eine Bindung bedeutet;
L Tyr, Q, Gly, Tyr(Me) oder eine Bindung bedeutet;
M Arg-OH, Arg-$NH_2$, OH, OR, $NH_2$, NHR′, Gly-Lys-Arg-OH oder Gly-Lys-Arg-$NH_2$ bedeutet;
R unverzweigtes $(C_1-C_6)$-Alkyl bedeutet
und
R′ -$[CH_2]_n$-$NH_2$ oder -$[CH_2]_n$-NH-C(NH)$NH_2$, wobei n ganzzahlig ist und für 3-8 steht, bedeutet sowie deren physiologisch verträgliche Salze, wobei Peptide der Formel III, die in EP-A 231 752 beschriebenen Verbindungen sowie deren Salze, rat- [$Mac^{105}$]ANF(101-126), rat-[$Mpr^{105}$]ANF(101-126), rat-[$Mbu^{105}$]ANF-(101-126) und rat-[$Mpr^{105}$, D-$Ala^{107}$]ANF(105-126) ausgenommen sind.

Besonders bevorzugt sind Peptide der Formel I, worin

X Wasserstoff, Arginyl oder Arginyl-Arginyl, Ser, Q oder Ser-Ser bedeutet, wobei Q und Ser jeweils in der L- oder D-Konfiguration vorliegen können und die N-terminale Aminogruppe des Aminosäure- oder Dipeptid-Restes frei vorliegt oder durch Arginyl oder Arginyl-Arginyl acyliert ist oder X fehlen kann;
V Cys oder, falls X fehlt, Mercaptopropionsäure bedeutet;
A Phe bedeutet;
B Arg oder Lys bedeutet;
C Ile, Leu, Val oder einen L-2-Thienylalanin-Rest bedeutet;

D Asp, Gln, Leu, Ile, Asp($OBu^t$), Glu($OBu^t$), Tyr($Bu^t$) oder Tyr(Me) bedeutet;
E Gln, Thr oder Pro bedeutet;
F Ser oder Ala jeweils in ihrer L- oder D-Konfiguration bedeutet;
G Gly, Ala, oder D-Ala bedeutet und
Z für einen Rest oder Formel II steht
worin H Asn, Pro oder Thr, jeweils in ihrer L- oder D-Form, oder Gly bedeutet;
I Ser, Thr oder Ala jeweils in ihrer L- oder D-Form bedeutet;
J Phe, Tyr(Me), Cyclohexylalanyl oder einen 2-Thienylalanin-Rest bedeutet;
K Arg, Lys oder eine Bindung bedeutet;
L Tyr, Tyr($Bu^t$) oder eine Bindung bedeutet;
M OH, $NH_2$, NHR′, Gly-Lys-Arg-OH oder Gly-Lys-Arg-$NH_2$ bedeutet
und
R′ - $[CH_2]_2$-$NH_2$ oder -$[CH_2]_n$-NH-C(NH)$NH_2$ bedeutet,
wobei n ganzzahlig ist und für 3-8 steht;
sowie deren physiologisch verträgliche Salze, wobei Peptide der Formel III, die in EP-A 231 752 beschriebenen Verbindungen sowie deren Salze, rat-[$Mac^{105}$]ANF(101-126), rat-[$Mpr^{105}$]ANF(101-126), rat-[$Mbu^{105}$]ANF(101-126) und rat-[$Mpr^{105}$, D-$Ala^{107}$]ANF(105-126) ausgenommen sind.

Falls im Einzelfall nicht anders angegeben, kann Alkyl geradkettig oder verzweigt sein. Entsprechendes gilt für davon abgeleitete Reste, wie Alkoxy, Aralkyl oder Alkanoyl.

($C_6$-$C_{12}$)-Aryl bedeutet vorzugsweise Phenyl, Naphthyl oder Biphenylyl. Entsprechend sind davon abgeleitete Reste, wie Aryloxy, Aralkyl oder Aroyl, zu formulieren. Falls nicht anders angegeben, steht die Abkürzung eines Aminosäurerestes ohne einen Stereodeskriptor für den Rest in der L-Form (vgl. Schröder, Lübke, The Peptides, Band I, New York 1965, Seiten xiii-xxix).

Als Salze kommen insbesondere Alkali- oder Erdalkalisalze, Salze mit physiologisch verträglichen Aminen und Salze mit anorganischen oder organischen Säuren wie z.B. HCl, HBr, $H_2SO_4$, Maleinsäure, Fumarsäure in Frage.

Die Erfindung betrifft weiterhin ein Verfahren zur Herstellung von Peptiden der Formel I, das dadurch gekennzeichnet ist, daß man

a) ein Fragment mit C-terminaler freier Carboxylgruppe oder dessen aktiviertes Derivat mit einem entsprechenden Fragment mit N-terminaler freier Aminogruppe umsetzt oder

b) das Peptid stufenweise aufbaut, in der nach (a) oder (b) erhaltenen Verbindung gegebenenfalls eine oder mehrere zum Schutz anderer Funktionen temporär eingeführte Schutzgruppen abspaltet, eine Disulfidbrücke zwischen den beiden Cys-Resten knüpft, wobei die letztgennanten beiden Maßnahmen auch in umgekehrter Reihenfolge durchgeführt werden können, und die so erhaltenen Verbindung der Formel I gegebenenfalls in ihr physiologisch verträgliches Salz überführt.

Die Peptide der vorliegenden Erfindung wurden nach allgemein bekannten Methoden der Peptidchemie, siehe z.B. Houben-Weyl, Methoden der organischen Chemie, Band 15/2, bevorzugt mittels Festphasensynthese wie z.B. von B. Merrifield, J.Am.Chem.Soc. 85, 2149 (1983) oder R. C. Sheppard Int.J.Peptide Protein Res. 21, 118 (1963) beschrieben oder äquivalente bekannte Methoden hergestellt. Als α-Aminoschutzgruppe werden Urethanschutzgruppen wie z.B. die tert.-Butyloxycarbonyl(Boc)- oder Fluorenylmethyloxycarbonyl(Fmoc)-Schutzgruppe verwendet. Falls zur Verhinderung von Nebenreaktionen oder für die Synthese spezieller Peptide erforderlich sind die funktionellen Gruppen in der Seitenkette von Aminosäuren durch geeignete Schutzgruppen (siehe z.B. T.W.Greene, "Protective Groups in Organic Synthesis") zusätzlich geschützt, wobei in erster Linie Arg(Tos), Arg(Mts), Arg(Mtr), Asp(OBzl), Asp($OBu^t$), Cys (4-MeBzl), Cys(Acm), Cys(SBut), Glu(OBzl), Glu-(OBut), His(Tos), His(Fmoc), Hios(Dnp), His(Trt), Lys(Cl-2), Lys (Boc), Met(O), Ser(Bzl), Ser(But), Thr(Bzl), Thr($Bu^t$), Trp(Mts), Trp(CHO), Tyr(Br-Z), Tyr(Bzl) oder Tyr($Bu^t$) eingesetzt werden.

Die Festphasensynthese beginnt am C-terminalen Ende des Peptids mit der Kupplung einer geschützten Aminosäure an ein entsprechendes Harz. Derartige Ausgangsmaterialien können durch Verknüpfung einer geschützten Aminosäure mit einem mit einer Chlormethyl-, Hydroxymethyl-, Benzhydrylamino-(BHA)-, Methylbenzhydrylamino(MBHA)-Gruppe modifiziertem Polystyrol- oder Polyacrylamid-Harz über eine Ester- bzw. Amidbindung erhalten werden. Die als Trägermaterial verwendeten Harze sind kommerziell erhältlich. BHA- und MBHA-Harze werden für gewöhnlich verwendet, wenn das synthetisierte Peptid am C-Terminus eine freie Amidgruppe enthalten soll. Falls das Peptid eine sekundäre Amidgruppe am C-terminalen Ende enthalten soll wird ein Chlormethyl- bzw. Hydroxymethyl-Harz verwendet und die Abspaltung mit den entsprechenden Aminen durchgeführt. Will man z.B. das Ethylamid erhalten, kann das Peptid mit Ethylamin vom Harz abgespalten werden, wobei die Abspaltung der Seitenketten-Schutzgruppen nachfolgend durch andere geeignete Reagenzien, erfolgt. Sollen in Peptid die tert.-Butyl-Schutzgruppen der

Aminosäure Seitenkette erhalten bleiben, so wird die Synthese mit der Fmoc-Schutzgruppe zur temporären Blockierung der α-Amino-Gruppe der Aminosäure unter Verwendung der z.B. bei R.C. Sheppard, J.Chem.Soc., Chem.Comm. 1982, 587 beschriebenen Methodik durchgeführt, wobei die Guanidino-Funktion des Arginins durch Protonierung mit Pyridinium-Perchlorat geschützt wird und der Schutz der anderen in der Seitenkette funktionalisierten Aminosäuren mit durch katalytische Transferhydrierung (A. Felix et al. J. Org. Chem. 13, 4194 (1978)) oder durch Natrium in flüssigem Ammoniak (W.Roberts, J.Am.Chem.Soc. 76, 6103 (1954)) abspaltbaren Benzylschutzgruppen erfolgt.

Nach Abspaltung der Aminoschutzgruppe der an das Harz gekuppelten Aminosäure mit einem geeigneten Reagenz, wie z.B. Trifluoressigsäure in Methylenchlorid im Falle der Boc-Schutzgruppe oder einer 20 %igen Lösung von Piperidin in Dimethylformamid im Falle der Fmoc-Schutzgruppe, werden die nachfolgend geschützten Aminosäuren nacheinander in der gewünschten Reihenfolge aufgekuppelt Die intermediär entstehenden N-terminal geschützten Peptidharze werden vor der Verknüpfung mit dem nachfolgenden Aminosäurederivat durch die vorbeschriebenen Reagenzien entblockiert.

Als Kupplungsreagenz können alle möglichen in der Peptidsynthese verwendeten Aktivierungs-Reagenzien, siehe z.B. Houben-Weyl, Methoden der organischen Chemie, Band 15/2, verwendet werden, insbesondere aber Carbodiimide wie z.B. N,N′-Dicyclohexylcarbodiimid, N,N′-Diisopropylcarbodiimid oder N-Ethyl-N′-(3-dimethylaminopropyl)carbodiimid. Die Kupplung kann dabei direkt durch Addition von Aminosäurederivat mit dem Aktivierungsreagenz und gegebenenfalls einem die Racemisierung unterdrückenden Zusatz wie z.B. 1-Hydroxybenzotriazol (HOBt) (W. König, R. Geiger, Chem. Ber. 103, 708 (1970)) oder 3-Hydroxy-4-oxo-3,4-dihydrobenzotriazin (HOObt) (W. König, R.Geiger, Chem.Ber. 103, 2054 (1970)) zum Harz durchgeführt werden oder aber die Voraktivierung des Aminosäurederivats als symmetrisches Anhydrid oder HOBt- bzw. HOObt-Ester kann separat erfolgen und die Lösung der aktivierten Spezies in einem geeigneten Lösungsmittel zum kupplungsfähigen Peptidharz gegeben werden.

Die Kupplung bzw. Aktivierung der Aminosäurederivate mit einem der oben genannten Aktivierungsreagenzien kann in Dimethylformamid oder Methylenchlorid oder einer Mischung aus beiden durchgeführt werden. Das aktivierte Aminosäurederivat wird üblicherweise in einem 1,5 bis 4 fachen Überschuß eingesetzt. In Fällen, in denen eine unvollständige Kupplung eintritt, wird die Kupplungsreaktion wiederholt, ohne vorher die für die Kupplung der nächstfolgenden Aminosäure nötige Entblockierung der α-Aminogruppe des Peptidharzes durchzuführen.

Der erfolgreiche Ablauf der Kupplungsreaktion kann mittels der Ninhydrin-Reaktion, wie z.B. von E.Kaiser et al. Anal. Biochem. 34 595 (1970) beschrieben, überprüft werden. Die Synthese kann auch automatisiert z.B. mit einem Peptid-Synthesizer Modell 430A der Fa. Applied Biosystems durchgeführt werden, wobei entweder die vom Gerätehersteller vorgesehenen Syntheseprogramme oder aber auch vom Benutzer selbst erstellte verwendet werden können. Letztere werden insbesondere bei der Verwendung von mit der Fmoc-Gruppe geschützten Aminosäurederivaten eingesetzt.

Nach Synthese der Peptide in der vorgehend beschriebenen Weise kann das Peptid vom Harz mit Reagenzien, wie z.B. flüssigem Fluorwasserstoff (bevorzugt bei den nach der Boc-Methode hergestellten Peptiden) oder Trifluoressigsäure (bevorzugt-bei den nach der Fmoc-Methode synthetisierten Peptiden) abgespalten werden. Diese Reagenzien spalten nicht nur das Peptid vom Harz sondern auch die weiteren Seitenkettenschutzgruppen der Aminosäurederivate. Auf diese Weise erhält man außer bei der Verwendung von BHA- und MBHA-Harzen das Peptid in Form der freien Säure. Bei den BHA- bzw. MBHA-Harzen erhält man bei der Spaltung mit Fluorwasserstoff oder Trifluormethansulfonsäure das Peptid als Säureamid. Weitere Verfahren zur Herstellung von Peptidamiden sind in den deutschen Patentanmeldungen P 37 11 866.8 und P 37 43 620.1 beschrieben. Hier erfolgt die Abspaltung der Peptidamide vom Harz durch Behandlung mit in der Peptidsynthese üblicherweise verwendeten mittelstarken Säuren (z.B. Trifluoressigsäure) wobei als Kationenfänger Substanzen wie Phenol, Kresol, Thiokresol, Anisol, Thioanisol, Ethandithiol, Dimethylsulfid, Ethylmethylsulfid oder ähnliche in der Festphasensynthese übliche Kationenfänger einzeln oder eine Mischung von zwei oder mehr dieser Hilfsmittel zugesetzt werden. Die Trifluoressigsäure kann dabei auch durch geeignete Läsungsmittel, wie z.B. Methylenchlorid, verdünnt angewendet werden.

Bei der Abspaltung mit Fluorwasserstoff, Trifluormethansulfonsäure und Trifluoressigsäure werden als Kationenfänger üblicherweise Substanzen, wie Phenol, Kresol, Thiokresol, Thioanisol, Dimethylsulfid, Ethylmethylsulfid oder eine Mischung von zwei oder mehr dieser Hilfsmittel zugesetzt. Die Trifluoressigsäure kann dabei auch durch geeignete Lösungsmittel, wie z.B. Methylenchlorid verdünnt angewendet werden. Falls die tert.-Butyl bzw. Benzylseitenketten-Schutzgruppen der Peptide erhalten bleiben sollen, wird die Abspaltung des an einem besonders modifizierten Trägerharz synthetisierten Peptides mit 1 % Trifluoressigsäure in Methylenchlorid durchgeführt, wie z.B. bei R.C. Sheppard J.Chem.Soc., Chem.Comm. 1982, 587 beschrieben. Sollen einzelne tert.-Butyl bzw. Benzylseitenketten-Schutzgruppen erhalten bleiben, so verwendet man eine geeignete Kombination der Synthese- und Abspaltmethoden.

Für die Synthese von Peptiden mit einer C-terminalen Amidgruppierung oder einer ω-Amino- bzw. ω-Guanidinoalkylgrupierung wird ebenfalls das von Sheppard beschriebene modifizierte Trägerharz verwendet. Nach der Synthese wird das in der Seitenkette vollgeschützte Peptid vom Harz abgespalten und anschließend in klassischer Lösungssynthese mit dem entsprechenden Amin bzw. ω-Aminoalkylamin oder ω-Guanidinoalkylamin umgesetzt, wobei gegebenenfalls vorhandene weitere funktionelle Gruppen in bekannter Weise temporär geschützt werden können.

Ein weiteres Verfahren zur Herstellung von Peptiden mit einer ω-Aminoalkylgruppierung ist in der deutschen Patentanmeldung P 36 35 670.0 beschrieben.

Die Peptide der vorliegende Erfindung wurden vorzugsweise unter Verwendung der Festphasentechnik nach zwei generellen Schutzgruppentaktiken synthetisiert:
Die Synthese erfolgte mit einem automatischen Peptidsynthesizer Modell 4300 der Fa. Applied Biosystems unter Verwendung von Boc- bzw. Fmoc-Schutzgruppen zur temporären blockierung der α-Aminogruppe.
Bei Verwendung der Boc-Schutzgruppe wurden für die Synthese die vom Hersteller des Gerätes vorprogrammierten Synthesezyklen benutzt.
Die Synthese der Peptide mit einer freien Carboxylgruppe am C-terminalen Ende erfolgte an einem mit der entsprechenden Boc-Aminosäure funktionalisiertem 4-(Hydroxymethyl)phenylacetamidomethylpolystyrolharz (R.B. Merrifield, J. Org. Chem. 43, 2845 (1978)) der Fa. Applied Biosystems. Für die Herstellung der Peptidamide wurde ein MBHA-Harz derselben Firma verwendet.
Als Aktivierungsreagenzien dienten N,N′-Dicyclohexylcarbodiimid oder N,N′-Diisopropylcarbodiimid. Die Aktivierung erfolgte als symmetrisches Anhydrid oder als HOBt-Ester in $CH_2$-$Cl_2$ oder $CH_2Cl_2$- DMF-Gemischen oder DMF. Für die Kupplung wurden 2-4 Äquivalente an aktiviertem Aminosäurederivat eingesetzt. Für Fälle in denen die Kupplung unvollständig verlief, wurde die Reaktion wiederholt.
Der Verknüpfung der beiden Cys-Reste durch eine Disulfidbrücke erfolgt vorzugsweise nach einer der in "Perspectives in Peptide Chemistry", Karger Basel 1981, Seiten 31-44 oder Schröder, Lübke, "The Peptides", Volume I, Academic Press, New York, London 1965, Seiten 235-239 beschriebenen Methoden. Bevorzugt ist die Oxydation mit Luft oder $J_2$.

Bei der Verwendung der Fmoc-Schutzgruppe zum temporären Schutz der α-Aminogruppe wurden für die Synthese mit dem automatischen Peptid-Synthesizer Modell 430A der Fa. Applied Biosystems eigene Syntheseprogramme eingegeben. Die Synthese erfolgte an einem p-Benzyloxybenzylalkohol-Harz (S. Wang, J.Am.Chem.Soc. 95, 1328 (1973)) der Fa. Bachem das nach bekannter Methode (E. Atherton et al. J.C.S.Chem. Comm. 1981, 336) mit der entsprechenden Aminosäure verestert war. Die Aktivierung der Aminosäurederivate als HOBt-Ester erfolgte direkt in den vom Gerätehersteller gelieferten Aminosäurecartridges durch Zugabe einer Lösung von Diisopropylcarbodiimid in DMF zu der vorher eingewogenen Mischung aus Aminosäurederivat und HOBt oder HOOBt. Ebenfalls eingesetzt werden können in Substanz hergestellte Fmoc-Aminosäure-OObt Ester wie sie in der europäischen Patentanmeldung 87107634.5 beschrieben sind. Die Abspaltung der Fmoc-Schutzgruppe erfolgte mit einer 20 %igen Lösung von Piperidin in DMF im Reaktionsgefäß. Der verwendete Überschuß an reaktivem Aminosäurederivat betrug 1,5 bis 2,5 Äquivalente. Falls die Kupplung nicht vollständig war wurde sie wie bei der Boc-Methode wiederholt.

Um die Glycosylreste in Serin bzw. Threonin einzuführen, müssen vorher die Aminogruppe und die Carboxylgruppe geeignet geschützt sein. Als besonders günstig erwiesen sich hierbei Schutzgruppen, die durch katalytische Hydrierung oder durch sekundäre Amine abspaltbar sind. Im ersten Fall sind dies Schutzgruppen vom Benzyl-Typ, wie z.B. der Benzyloxycarbonyl(Z-) oder p-Nitro-benzyloxycarbonyl-Rest als Aminoschutzgruppen und der Benzyl-(-OBzl) oder p-Nitrobenzylester für die Carboxylgruppe. Mit sekundären Aminen läßt sich der 9-Fluorenyl-methyloxycarbonyl-(Fmoc-)Rest abspalten. Besonders günstig erwies sich die Verwendung von Fmoc-L- bzw. Fmoc-D-Ser-Obzl, da nach Glycosylierung die Benzylreste der entsprechenden Fmoc-L- bzw. Fmoc-D-Ser($R^1$)-OBzl durch katalytische Hydrierung unter Erhalt der Fmoc-Gruppe selektiv abgespalten werden konnten. Das ist besonders überraschend, da in letzter Zeit immer wieder berichtet wurde, daß der Fmoc-Rest durch katalytische Hydrierung abgespalten wird [z.B. von R. Geiger und W. König in E. Gross und J. Meienhofer (Eds): The Peptides, Vol. 3, p. 24, Academic Press, 1981].

Bei der Synthese der O-Glycosyl-Aminosäure-Bausteine sind zwei polyfunktionelle Reaktionspartner (Kohlen hydrat und Serin bzw. Threonin) zu verknüpfen. Beide müssen sich selektiv blockieren und deblockieren lassen. In der Glycosylkomponente muß das anomere Zentrum freisetzbar und funktionalisierbar sein und in der Aminosäurekomponente darf nur die für die Verknüpfung notwendige Hydroxylgruppe deblockiert sein. Je nach dem Typ der angestrebten glycosidischen Bindung (1,2-cis- oder 1,2-trans-Glycoside) ist es notwendig, geeignete Schutzgruppen für die Blockierung der Hydroxy- oder Aminogruppen in die Glycosylkomponente einzuführen sowie Reaktionsbedingungen für den Verknüpfungsschritt auszuarbeiten, der stereoselktiv zu nur einem der beiden möglichen Anomeren führt.

Für die Herstellung der erfindungsgemäßen Verbindungen der Formel I, die einen O-Glycosylrest enthalten, werden sowohl die in der Literatur bekannten, meist natürlichen O-Glycosylaminosäure-Bausteine wie z.B. von K. Dill et al. [Carbohydr. Res. 123 (1983) 137-144], H. Kunz [Nach. Chem. Tech. Lab. 32 (1984) 11] und H. Paulsen [Chem. Soc. Res. 13 (1) (1984) 25-45] beschrieben, als auch die artificiellen O-Glycosylserin- bzw. Glycosylthreonin-Derivate, die nach den in der Kohlenhydratchemie üblichen, wie von A.F. Bochkov und G.E. Zaikov [Chemistry of the O-glycosidic bond, Pergamon Press 157 (1979)], H. Paulsen [Angew. Chem. 94 (1982) 184-201] und R.R. Schmidt [Angew. Chem. 98 (1986) 213-236] beschriebenen oder modifizierten Glycosylierungsverfahren hergestellt werden, verwendet. Die Einführung der glycosylierten Aminosäure-Bausteine erfolgt am cyclisierten Peptid.

Die erfindungsgemäßen Peptide sind Analoga des ANF (Atrial Natriuretic Factor), einem Peptid, das im Vorhof von Säugerherzen gebildet wird und natriuretische, diuretische und vasoaktive Wirkung hat (Currie et al. Science 223, 67 1984: Kangawa, Matsuo, Biochem. Biophys. Res. Commun. 118, 131 1984). Durch die erfindungsgemäßen Modifikationen kön nen die Wirkungen des ANF verstärkt oder spezifisch verändert werden. So läßt sich zum Beispiel die Wirkdauer und das Wirkprofil der natriuretischen Eigenschaften positiv beeinflussen oder die vasoaktive Wirkung zurückdrängen oder auch der entgegengesetzte Effekt erzielen. Weiterhin zeigen die erfindungsgemäßen Peptide immunmodulierende Eigenschaften, die bei den aus Vorhöfen von Säugerherzen isolierten Peptiden noch nicht vorbeschrieben waren.

In dem nachfolgend beschriebenen Rezeptor-Bindungs-Test wird die für die biologische Wirkung der erfindungsgemäßen Peptide notwendige Bindungsstärke untersucht.

**Präparation von Rinder-Nebennierencortex-Membranen**

Frische Rindernebennieren wurden vom Schlachthof bezogen und auf Eis transportiert. Nach der Entfernung des Nebennierenmarks wurde die Rinde in eiskaltem Puffer A (5 mmol/l TRIS, 1 mmol/l $MgCl_2$, 250 mmol/l sucrose, pH 7,4, bei 0 °C) mit einer Schere kleingeschnitten. Die Homogenisierung des Gewebes wurde mit einem ®Waring Blender in Puffer A durchgeführt. Größere Partikel wurden durch anschließendes Filtrieren durch ein Gazetuch abgetrennt und das Filtrat mit einem Potter Homogenisator mit 20 Bewegungen nachhomogenisiert. Danach wurde mit einer 10-minütigen Zentrifugation bei 3000 x g und 4 °C die Zellpartikel höherer Dichte abgetrennt und verworfen. Die Plasmamembranen im Überstand wurden anschließend bei 39000 x g über 10 Minuten abgetrennt und in Puffer B (75 mmol/l TRIS, 25 mmol/l $MgCl_2$, pH 7,4 bei 4 °C) resuspendiert. Nach einer zweimaligen Wiederholung dieses Zentrifugationsschrittes wurden die Plasmamembranen in Puffer B mit 250 mmol/l Sucrose aufgenommen und portionsweise, entsprechend 1 g ursprünglichem Cortexgewebe/ml in flüssigem Stickstoff eingefroren. Bei der weiteren Lagerung bei -70 °C wurde über mehrere Monate kein Abfall der Bindungsaktivität festgestellt.

**Rezeptorbindung**

Für die Bindungsexperimente wurde die Membranen aufgetaut, 10 Minuten bei 39000 x g zentrifugiert und in Puffer C (100 mmol/l NaCl, 0,1 mmol/l EDTA-$Na_2$, 50 mmol/l HEPES, pH 7,4, und 0,2 % Rinderserumalbumin, um Wandadsorption zu reduzieren) suspendiert. Der Plasmamembransuspension wurden 40 µmol/l Aprotinin, ein Peptidaseinhibitor, zugefügt, um während der Inkubationszeit einen Abbau des radioaktiv markierten Liganden (Radioligand) zu verhindern. Das Bindungsexperiment wurde in Mikrotiterplatten bei 25 °C 60 Minuten bis zum Equilibrium durchgeführt. Gestartet wurde die Bindung durch Zugabe der Membransuspension. Das Endvolumen pro Probe betrug 250 µl. Darin enthalten waren ca. 14000 cpm des Radioliganden ($^{125}$J-human-Atriales Natriuretisches Peptid$_{1-28}$ von Amersham Buchler, FRG) mit einer spezifischen Aktivität von 74 TBq/mmol und ein Anteil des Membranproteins, welches etwa 50 % des zugegebenen Radioliganden gebunden hat. Freier und gebundener Radioligand wurden durch schnelle Filtration über einen ®Whatman GF/C Glasfiber-Filter mittels eines ®Skatron cell harvester getrennt. Um die unspezifische Bindung zu reduzieren, wurden die Filter zuvor 1 Stunde in frisch angesetztem 3 %igen Polyethylenimin bei pH 10 eingelegt. Nach der Filtration wurde die auf den Filtern retinierte Menge des Radioliganden in einem Gamma-Szintillationszähler gemessen. Die unspezifische Bindung wurde als Bindung in Anwesenheit von 0,5 µmol/l Atriopeptin III (H-Ser-Ser-Cys-Phe-Gly-Gly-Arg-Ile-Asp-Arg-Ile-Gly-Ala-Gln-Ser-Gly-Leu-Gly-Cys-Asn-Ser-Phe-Arg-Tyr-OH) definiert, eine Menge die für eine komplete Verdrängung des Radioliganden von den Rezeptoren ausreicht.

**Berechnung der Daten**

Die $IC_{50}$-Werte stellen die Konzentration der Testsubstanz dar, die notwndig ist, um 50 % der Bindung des Radioli ganden zu hemmen. Die Werte wurden mit einem Mikrocomputer anhand der gemessenen Werte nach der Median-Effekt-Gleichung nach Chen (1976), J. Theor. Biol. 59, 253-276, berechnet.

| Bindungsdaten (Atriopeptin III-Verdrängung) | | |
|---|---|---|
| Beispiel-Nr. | $IC_{50}$[nm] Substanz | $IC_{50}$[nm] Atriopeptin III |
| 8 | 0,49 | 7,40 |
| 9 | 1,40 | 7,40 |
| 10 | 0,05 | 3,40 |
| 11 | 0,07 | 8,30 |
| 12 | 0,06 | 8,30 |
| 13 | 0,79 | 3,50 |
| 14 | 1,10 | 8,60 |
| 15 | 2,40 | 8,50 |
| 17 | 3,20 | 5,40 |
| 21 | 1,40 | 3,50 |
| 22 | 0,48 | 5,40 |

Die vasorelaxierende Wirkung der erfindungsgemäßen Peptide wurde in vitro an mit 25 mM KCl vorkontrahierten Aortenstreifen des Meerschweinchens getestet. Die diuretische und natriuretische Wirkung wurde in vivo durch i.v. Gabe an narkotisierte Ratten nachgewiesen. Als Vergleich wurde Atriopeptin III herangezogen.

Die Erfindung betrifft daher auch die Verwendung der Peptide der Formel I als Heilmittel und pharmazeutische Zubereitungen, welche diese Peptide enthalten. Bevorzugt ist die Anwendung am Menschen.

Die erfindungsgemäßen neuen Peptide wirken einzeln oder in Kombination diuretisch, salidiuretisch, vasorelaxierend und immunmodulierend oder können bei chronischer oder/und akuter Niereninsuffizienz sowie zur Protektion der Niere gegenüber nierentoxischen Substanzen, z.B. Cyclosporin, insbesondere bei Nierentransplantationen eingesetzt werden, in einer Dosis von 10 bis 20000 picamol/kg (25 ng - 50 µg/kg). Die Peptide können dabei parenteral (i.v., s.c., i. m. oder auch durch Infusion in die arteria renalis) in einem physiologisch verträglichen Medium verabreicht werden.

Zur parenteralen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol oder Glycerin, daneben auch Zukkerlösungen wie Glucose- oder Mannitlösungen, oder auch eine Mischung aus den verschiedenen genannten Lösungsmitteln.

Ebenso können die Wirkstoffe durch Implantate z.B. aus Polylactid, Polyglycolid, Copolymerisate aus Milch- und Glykolsäure oder Poly-3-hydroxybuttersäure bzw. intranasale Zubereitungen appliziert werden. Bei der intranasalen Applikation muß wegen der schlechteren Resorption die Dosis verzehnfacht werden. Ferner können die Peptide in Form ihrer physiologisch verträglichen Salze oder Metallkomplexe verabreicht werden.

Die Erfindung betrifft wieterhin die Verwendung von Verbindungen der Formel I bei der Behandlung des Glaukoms und/oder der Herabsetzung des Augeninnendrucks bei Säugern, vorzugsweise beim Menschen durch deren topische oder systemische Anwendung.

Bei der ophthalmologischen Anwendung der erfindungsgemäßen Verbindungen werden diese zweckmäßig in üblicher Weise in pharmazeutische Präparate eingearbeitet. Sie können in die üblichen Verabreichungsformen, wie Lösungen, Salben, Emulsionen und auch in Depot-Form gebracht werden. Der Wirkstoff kann gegebenenfalls auch in mikroverkapselter Form vorliegen. Die Präparate können verträgliche, organische oder anorganische Begleitstoffe, beispielsweise Suspendirmittel, Lösungsmittel, antibakterielle Mittel,

Netzmittel und Konservierungsmittel enthalten. Topische Anwendungsformen werden bevorzugt. Auch parenterale Zubereitungen können angewendet werden. Bezüglich systemischer Anwendungsformen werden etwa 25 ng - 5 µg/kg ein- bis dreimal täglich verabreicht. Für die topische Anwendung werden vorzugsweise Lösungen, Salben oder ophthalmische inserts ("solid inserts") benutzt. Topische Anwendungsformen können 0,001 - 5 Gewichtsprozente des Wirkstoffs enthalten. Höhere oder niedrigere Dosierungen können ebenfalls angewendet werden, vorausgesetzt sie erniedrigen den Augeninnendruck. Vorzugsweise werden am menschlichen Auge 0,01 mg - 1 mg des Wirkstoffs angewendet. Für die bevorzugte topische Anwendung am Auge werden die Verbindungen der Formel I in Kombination mit physiologisch verträglichen Trägerstoffen, wie z.B. wäßrige Methylcellulose appliziert. Die Kombination kann in Form einer Suspension, Lösung, Salbe, Emulsion oder eines Okusert bestehen. Eine bevorzugte Kombination ist die, die die Penetration der Wirkstoffe in das Auge erleichtert. Eine weitere bevorzugte topische Anwendungsform ist die Kombination der Verbindungen der Formel I mit Verbindungen, wie z.B. Benzalkomiumchlorid, welches die Penetration des Wirkstoffs in das Auge erleichtert. Verbindungen der Formel I können auch in Kombination mit anderen Antiglaucoma-Verbindungen zur Behandlung dr Glaukomkrankheit eingesetzt werden.

Die topischen Trägerstoffe können organische oder anorganische Verbindungen sein. Typische phamazeutische ge brauchte Trägerstoffe sind wäßrige Lösungen, die z.B. Puffersysteme oder isotonische Mischungen von Wasser und wassermischbaren Lösungsmitteln sind, wie z.B. Alkohole oder Arylalkohole, Öle, Polyalkylenglykole, Ethylcellulose, Carboxymethylcellulose, Polyvinylpyrrolidon oder Isopropylmyristat. Geeignete Puffersubstanzen sind z.B. Natriumchlorid, Natriumborat, Natriumphosphat, Natriumacetat oder auch Gluconatpuffer. Die topische Anwendungsform kann auch nicht toxische Hilfsstoffe enthalten wie z. B. emulgierende Konservierungsstoffe, Vernetzer, wie z. B. Polyethylenglykole, antibakterielle Verbindungen, wie z.B. quaternäre Ammoniumverbindungen, Benzalkoniumchlorid, Phenylquecksilbersalze, Benzylalkohol, Phenylethanol, Triethanolaminoleate, Thiosorbitol und weitere ähnliche Stoffe, die in topischen ophthalmischen Zubereitungen gebraucht werden. Die topischen Anwendungsform kann ebenso in Form eines ophthalmischen inserts ("solid insert") bestehen. Hierzu kann z.B. ein festes wasserlösliches Polymer als Carrier für den Wirkstoff gebraucht werden. Als Polymer kann jedes wasserlösliche, nicht toxische Polymer benutzt werden, wie z.B. Cellulosederivate, wie z.B. Methylcellulose, Natriumcarboxymethylcellulose, ($C_1$-$C_6$)-Hydroxyalkylcellulose, wie z.B. Hydroxyethylcellulose, Hydroxypropylcellulose und Hydroxypropylmethylcellulose, Acrylsäurederivate, wie Polyacrylsäuresalze, Ethylacrylate und Polyacrylamide. Weiterhin können z.B. Gelatine, Stärkederivate, Alginate, Pektine, Polyvinylalkohole, Polyvinylpyrrolidone, Polyvinylmethylether, Polyethylenoxide oder auch Mischungen von den verschiedenen Polymeren benutzt werden. Solid inserts, die für eine topische Anwendung in Frage kommen, beschreibt die britische Patentanmeldung 1524405.

<u>Verzeichnis der Abkürzungen:</u>

Die für Aminosäuren verwendeten Abkürzungen entsprechen dem in der Peptidchemie üblichen drei Buchstaben-Code wie er z.B. in Europ. J. Biochem. <u>138</u>, 9 (1984) beschrieben ist. Weitere verwendete Abkürzungen sind nachfolgend aufgelistet.

| | |
|---|---|
| Acm | Acetamidomethyl |
| ε-Ahx | ε-Aminohexanoyl |
| Aoc | cis, endo-2-Azabicyclo[3.3.0]octan-3-S-carbonyl |
| Boc | tert.-Butyloxycarbonyl |
| But | tert.-Butyl |
| Bzl | Benzyl |
| Cl-Z | 4-Chlor-benzyloxycarbonyl |
| DMF | Dimethylformamid |
| Dnp | 2,4-Dinitrophenyl |
| Fmoc | 9-Fluorenylmethyloxycarbonyl |
| Mac | Mercaptoessigsäure |
| Mbu | Mercaptobuttersäure |
| Me | Methyl |
| 4-Mebzl | 4-Methylbenzyl |
| Mpr | Mercaptopropionsäure |
| Mtr | 4-Methoxy-2,3,6-trimethylphenylsulfonyl |
| Mts | Mesitylen-2-sulfonyl |

TFA Trifluoressigsäure

Tcs 4-Methylphenylsulfonyl

Trt Trityl

Die folgenden Beispiele sollen die bevorzugten Methoden zur Festphasen-Synthese der erfindungsgemäßen Peptide verdeutlichen, ohne daß die Erfindung darauf eingeschränkt wäre.

Referenzbeispiel 1:

Die Synthese des natriuretisch, diuretisch und vasoaktiv wirksamen ANF-Derivats mit der Formel

```
                                                                  13
H-Ser-Ser-Cys-Phe-Gly-Gly-Arg-Ile-Glu-Arg-Ile-Gly-Ala-
           L_____________________________________
 14                                                   24
-Gln-Ser-Gly-Leu-Gly-Cys-Asn-Ser-Phe-Arg-Tyr-OH
```

wurde mit einem automatischen Peptid-Synthesizer Modell 430A der Fa. Applied Biosystems nach der Boc-Methode an einem mit Boc-Tyr(Br-Z)-OH substituiertem 4-(Hydroxymethyl)phenylacetamidomethylpolystyrol-Harz der Fa. Applied Biosystems, das 0,5 mmol Tyrosin enthielt durchgeführt. Für die Synthese wurden folgende ebenfalls von Applied Biosystems gelieferte Aminosäurederivate verwendet, die für den Einsatz im Peptidsynthesizer schon zu je 2 mmol in Cartridges abgewogen waren: Boc-Arg(Tos)-OH, Boc-Phe-OH, Boc-Ser(Bzl)-OH, Box-Asn-OH, Boc-Cys(5-MeBzl)-OH, Box-Gly-OH, Boc-Leu-OH, Boc-Gln-OH, Boc-Ala-OH, Boc-Ile-OH, Boc-Glu(OBzl)-OH. Für die Synthese wurden die vom Gerätehersteller vorgesehenen Programme verwendet, wie exemplarisch nachfolgend aufgelistet.

1) 65 % Trifluoressigsäure/$CH_2Cl_2$, 5 ml, 2 min

2) 65 % Trifluoressigsäure/$CH_2Cl_2$, 5 ml, 15 min

3) Waschen mit $CH_2Cl_2$ (3mal 10 ml)

4) Waschen mit 10 % Diisopropylethylamin in DMF (2mal 10 ml)

5) Waschen mit DMF (3mal 10 ml)

6) Kupplung des als symmetrisches Anhydrid oder HOBt-Ester voraktivierten Aminosäurederivats (15-30 min), evtl. Wiederholung bei unvollständiger Kupplung

7) Waschen mit $CH_2Cl_2$ (6mal 10 ml)

Nach Beendigung des Synthese wurde das Peptid-Harz im Vakuum über Molekularsieb getrocknet. Zur Abspaltung des Peptids vom Harz wurden 1 g Peptid-Harz zusammen mit 0,5 g p-Kresol und 0,5 g p-Thiokresol in das Reaktionsgefäß einer HF-Apparatur der Fa. Protein Research Foundation, Minowa, Osaka, Japan eingewogen und nach Evakuieren der Apparatur ca. 10 ml flüssiges HF bei Trockeneistemperatur hinzudestilliert. Anschließend läßt man 1 h bei 0 °C reagieren und destilliert danach vorsichtig das überschüssige HF im Vakuum ab. Das zurückbleibende gelbrote Harz-Peptid-Gemisch wird mehrfach mit Essigester und tr. Ether gewaschen um die Kationenfänger zu entfernen.

Nach Trocknung wird das Peptid mit 30 %iger wäßriger Essigsäure vom Harz extrahiert und die erhaltene Rohpeptidlösung gefriergetrocknet.

Das gefriergetrocknete Rohpeptid wird an Sephadex® G25 superfine mit 1N Essigsäure von eventuell noch vorhandenem Kationenfänger abgetrennt und die peptidhaltigen Fraktionen weiter aufgearbeitet. Dazu wird das Rohpeptid in Trifluorethanol gelöst und mit einem 5 fachen Überschuß Tri-n-butylphosphin über Nacht reduziert. Nach Entfernen des Lösungsmittels im Vakuum wird der Rückstand mehrfach mit Essigester extrahiert und das als Niederschlag erhaltene Rohpeptid sofort nach kurzer Trocknung mit $I_2$ oxidiert. 0,5 mmol Peptid wird in 50 ml 90 %iger Essigsäure gelöst und diese Lösung schnell zu einer gut gerührten Lösung von 0,5 mmol $I_2$ und 1 mmol Natriumacetat in 450 ml 80 %iger wäßriger Essigsäure getropft. Nach 10 min Nachrühren wird die Lösung durch Zugabe von 0,1 N wäßriger Ascorbinsäure entfärbt und auf ca. 10 ml eingeengt und sofort an Sephadex® G25 superfine mit 1N wäßriger Essigsäure als Eluent entsalzt. Die das Peptid enthaltenen Fraktionen wurden vereinigt und gefriergetrocknet. Die Überprüfung der Reinheit erfolgte durch HPLC an Vydac®$C_{18}$ bzw. Vydac®$C_4$-Säulen mit einem Gradienten von Acetonitril in 0,1 %iger wäßriger Trifluoressigsäure. Zur Hochreinigung wird an gleichem Trägermaterial mit gleichem Laufmittelgemisch eine präparative HPLC-Trennung durchgeführt.

Referenzbeispiel 2:

Das natriuretisch, diuretisch und vasorelaxierend wirksame Peptid mit der Formel

```
1                                                                  14
H-Ser-Ser-Cys-Phe-Gly-Gly-Lys-Ile-Asp-Arg-Ile-Gly-Ala-Gln-
            └──────────┐
15                                               24
-Ser-Gly-Leu-Gly-Cys-Asn-Ser-Phe-Arg-Tyr-OH
```

wurde mit einem Peptid-Synthesizer Modell 430A der Fa. Applied Biosystems unter Verwendung der Fmoc-Methode an einem mit Fmoc-Tyr($Bu^t$)-OH veresterten p-Benzyloxybenzylalkohol-Harz der Fa. Bachem (Beladung 0,40 mmol/g Harz) stufenweise aufgebaut. Es wurde 1 g dieses Harzes eingesetzt und die Synthese mit für die Fmoc-Methode modifizierten Synthese-Programmen durchgeführt.

Man verwendete folgende Aminosäurederivate: Fmoc-Arg(Mtr)-OH, Fmoc-Phe-OH, Fmoc-Ser($Bu^t$)-OH, Fmoc-Asn-OH, Fmoc-Cys (Acm)-OH, Fmoc-Gly-OH, Fmoc-Leu-OH, Fmoc-Gln-OH, Fmoc-Ala-OH, Fmoc-Ile-OH, Fmoc-Asp($OBu^t$)-OH und Fmoc-Lys(Boc)-OH. In die Cartridges des Synthesizers wurde je 1 mmol Aminosäurederivat zusammen mit 1,5 - 2,5 Äquivalenten HOBt eingewogen. Die Aktivierung der Aminosäuren erfolgte direkt in den Cartridges durch Lösen in 4 ml DMF und Zugabe einer 0,55 molaren Lösung von N,N′-Diisopropylcarbodiimid in DMF (2 ml). Ein typischer Synthesezyklus ist nachfolgend aufgelistet:

1) Abspaltung der Fmoc-Gruppe mit 20 % Piperidin in DMF (zweimal 8 ml je 10 min)

2) Waschen mit DMF (6mal mit je 8 ml DMF)

3) Kupplung des in der Cartridge als HOBt-Ester voraktivierten Aminosäurederivats (25 - 45 min), evtl. Wiederholung bei unvollständiger Kupplung

4) Waschen mit DMF (6mal mit je 8 ml DMF)

Nach beendeter Synthese wurde vom Peptidharz zunächst die Fmoc-Schutzgruppe durch Behandlung mit 20 % Piperidin in DMF abgespalten und das gut mit DMF gewaschene Harz durch mehrfache Behandlung mit Isopropanol und Methyl-tert.-butylether geschrumpft. Nach Trocknung im Hochvakuum wurde das Peptid durch Rühren mit einer Mischung aus Trifluoressigsäure/$CH_2Cl_2$/Phenol (70:30:5), 4 h bei Raumtemperatur vom Harz abgespalten. Durch Nachbehandlung mit Trifluoressigsäure/Phenol/Dimethylsulfid (90:5:5) werden evtl. verbliebene Schtuzgrupppen abgespalten. Anschließend wird vom Harz abfiltriert, mit Abspaltlösung nachgewaschen und das Filtrat im Vakuum eingeengt. Durch mehrfaches Verrühren mit Essigester wurden die Kationenfänger entfernt. Nach Trocknung im Hochvakuum wurde das Rohpeptid mit 1N wäßrige Essigsäure an Sephadex® G25 superfine von evtl. noch vorhandenem Kationenfänger und Trifluoressigsäurespuren abgetrennt. Die peptidhaltigen Fraktionen wurden gefriergetrocknet und dann weiter aufgearbeitet. Die oxidative Abspaltung der Acm-Schutzgruppen des Cysteins erfolgte nach Kamber et al., Helv. Chim. Acta, 63, 899 (1980) in 90 %iger wäßriger Essigsäure mit einem 50-fachen Überschuß an $I_2$ bei einer Reaktionszeit von 15 min. Die Entsalzung und Endreinigung des so erhaltenen Rohpeptids erfolgte wie im Beispiel 1 beschrieben.

Referenzbeispiel 3:

Synthese von [des-$Tyr^{24}$, des-$Arg^{23}$]-r-Atriopeptin III-(4-amino)butylamid

Die Peptidsynthese erfolgt an 1 g des in der deutschen Patentanmeldung P 36 35 670.0 beschriebenen Harzes unter Verwendung vom Fmoc-Aminosäure-OObt-Estern mit einem automatischen Peptidsynthesizer Modell 430A der Fa. Applied Biosystems und selbst modifizierten Syntheseprogrammen.

Dazu werden jeweils 1 mMol des enstprechenden Aminosäurederivates in die vom Hersteller gelieferten Cartridges eingewogen, Fmoc-Arg(Mtr)-OH, Fmoc-Asn-OH und Fmoc-Gln-OH werden zusammen mit 1,5 mMol HOBt in die Cartridges eingewogen. Die Voraktivierung dieser Aminosäuren erfolgt direkt in den Cartridges durch Lösen in 4 ml DMF und Zugabe von 2 ml einer 0,55 M Lösung von Diisopropylcarbodiimid in DMF. Die HOObt-Ester werden in 6 ml DMF gelöst und dann ebenso wie die in situ voraktivierten Aminosäuren Arginin, Asparagin und Glutamin auf das vorher mit 20 % Piperidin in DMF entblockierte Harz gepumpt. Die in situ aktivierten Aminosäuren werden doppelt gekuppelt.

Nach beendeter Synthese wird das Peptid-butylamid unter gleichzeitiger Entfernung der Seitenkettenschutzgruppen mit Trifluoressigsäure, die Thioanisol und m-Kresol als Kationenfänger enthält, vom Harz abgespalten. Der nach Abziehen der Trifluoressigsäure erhaltene Rückstand wird mehrfach mit Essigester digeriert un zentrifugiert. Das verbliebene Rohpeptid wird zur Entfernung der Cysteinschutzgruppe mit Tributylphosphin in Trifluorethanol behandelt. Nach Entfernen des Lösungsmittels wird wiederum mit Essigester digeriert und zentrifugiert. Das reduzierte Rohpeptid wird sofort mit Jod in 80 %iger wäßriger Essigsäure oxidiert, der $I_2$-Überschuß mit Ascorbinsäure entfernt und die Reaktionsmischung nach Einengen auf ein kleines Volumen an ®Sephadex G25 mit wäßriger 1N Essigsäure entsalzt. Die das reine Peptid enthaltenden Fraktionen werden vereinigt und gefriergetrocknet.

Referenzbeispiel 4:

Synthese von

H-Aoc-Cys-Phe-D-Ala-Gly-Arg-Ile-Asp-Arg-Ile-Gly-Ala-Gln-Ser-Gly-Leu-Gly-Cys-Asn-Ser-Phe-Arg-Aoc-$NH_2$

Die Peptidsynthese erfolgt an 1 g des in der deutschen Patentanmeldung P 37 43 620.1 beschriebenen Harzes unter Verwendung von Fmoc-Aminosäure-OObt-Estern mit einem automatischen Peptidsynthesizer Modell 430A der Fa. Applied Biosystems und selbst modifizierten Syntheseprogrammen.

Dazu werden jeweils 1 mMol des entsprechenden Aminosäurederivates in die vom Hersteller gelieferten Cartridges eingewogen, Fmoc-Arg(Mtr)-OH, Fmoc-Asn-OH und Fmoc-Gln-OH werden zusammen mit 1.5 mMol HOBt in die Cartridges eingewogen. Die Voraktivierung dieser Aminosäuren erfolgt direkt in den Cartridges durch Lösen in 4 ml DMF und Zugabe von 2 ml einer 0,55 M Lösung von Diisopropylcarbodiimid in DMF. Die HOObt-Ester werden in 6 ml DMF gelöst und dann ebenso wie die in situ voraktivierten Aminosäuren Arginin, Asparagin und Glutamin auf das vorher mit 20 % Piperidin in DMF entblockierte Harz gepumpt. Die in situ aktivierten Aminosäuren werden doppelt gekuppelt.

Nach beendeter Synthese wird das Peptamid unter gleichzeitiger Entfernung der Seitenkettenschutzgruppen mit Trifluoressigsäure, die Thioanisol und m-Kresol als Kationenfänger enthält, vom Harz abgespalten. Der nach Abziehen der Trifluoressigsäure erhaltene Rückstand wird mehrfach mit Essigester digeriert und zentrifugiert. Das verbliebene Rohpeptid wird zur Entfernung der Cysteinschutzgruppe mit Tributylphosphin in Trifluorethanol behandelt. Nach Entfernen des Lösungsmittels wird wiederum mit Essigester digeriert und zentrifugiert. Das reduzierte Rohpeptid wird sofort mit Jod in 80 %iger wäßriger Essigsäure oxidiert, der $I_2$-Überschuß mit Ascorbinsäure entfernt und die Reaktionsmischung nach Einengen auf ein kleines Volumen an ®Sephadex G25 mit wäßriger 1N Essigsäure entsalzt. Die das reine Peptid enthaltenden Fraktionen werden vereinigt und gefriergetrocknet.

Folgende Verbindungen werden analog Referenzbeispiel 2 hergestellt

1. H-Ser-Ser-Cys-Phe-Gly-Gly-Arg-Ile-Asp-Arg-Ile-Gly-Ala-Gln-Ser-Gly-Leu-Gly-Cys-Asn-Ser-Tyr(Me)-Arg-Tyr-OH

2. H-Ser-Ser-Cys-Tyr(Me)-Gly-Gly-Arg-Ile-Asp-Arg-Ile-Gly-Ala-Gln-Ser-Gly-Leu-Gly-Cys-Asn-Ser-Phe-Arg-Tyr-OH

3. H-Ser-Ser-Cys-Phe-Gly-Gly-Arg-Ile-Asn-Arg-Ile-Gly-Ala-Gln-Ser-Gly-Leu-Gly-Cys-Asn-Ser-Phe-Arg-Tyr-OH

4. H-Ser-Ser-Cys-Phe-Gly-Gly-Arg-Ile-Asp-Arg-Pro-Gly-Ala-

Gln-Ser-Gly-Leu-Gly-Cys-Asn-Ser-Phe-Arg-Tyr-OH

5. H-Ser-Ser-Cys-Phe-Gly-Gly-Arg-Ile-Glu-Arg-Ile-Gly-Ala-

Pro-Ser-Gly-Leu-Gly-Cys-Thr-Ser-Phe-Arg-Tyr-OH

6. H-Cys-Phe-D-Ala-Gly-Arg-Ile-Asp-Arg-Cyclohexylalanyl-

Gly-Ala-Gln-Ser-Gly-Leu-Gly-Cys-Asn-Ser-Phe-Arg-Tyr-OH

7. H-Ser-Ser-Cys-Phe-NH-$(CH_2)_5$-CO-Arg-Ile-Asp-Arg-Ile-Gly-Ala-Gln-Ser-Gly-Leu-Gly-Cys-Asn-Ser-Phe-Arg-Tyr-OH

Folgende Verbindungen werden wie in Referenzbeispiel 2 hergestellt, wobei die erste Aminosäure an MBHA-Harz gekuppelt wird und die Abspaltung wie in Referenzbeispiel 1 beschrieben, mit Fluorwasserstoff oder Trifluormethansulfonsäure erfolgt.

8. H-Aoc-Cys-Phe-D-Ala-Gly-Arg-Ile-Asp-Arg-Ile-Gly-Ala-Gln-Ser-Gly-Leu-Gly-Cys-Asn-Ser-Phe-Lys-$NH_2$

9. H-D-Aoc-Cys-Phe-D-Ala-Gly-Arg-Ile-Asp-Arg-Ile-Gly-Ala-Gln-Ser-Gly-Leu-Gly-Cys-Asn-Ser-Phe-Lys-$NH_2$

10. H-Aoc-Cys-Phe-D-Ala-Gly-Arg-Ile-Asp-Arg-Ile-Gly-Ala-Gln-Ser-Gly-Leu-Gly-Cys-Asn-Ser-Phe-Arg-Aoc-$NH_2$

11. H-Aoc-Cys-Phe-D-Ala-Gly-Arg-Ile-Asp-Arg-tert.Butylglycyl-

Gly-Ala-Gln-Ser-Gly-Leu-Gly-Cys-Asn-Ser-Phe-Arg-$NH_2$

12. H-Aoc-Cys-Phe-D-Ala-Gly-Arg-Ile-Asp-Arg-Neopentylglycyl-

Gly-Ala-Gln-Ser-Gly-Leu-Gly-Cys-Asn-Ser-Phe-Arg-$NH_2$

13. H-Aoc-Cys-Phe-Gly-Gly-Arg-Ile-Asp-Arg-Ile-Gly-Ala-Gln-Ser-Gly-Leu-Gly-Cys-Asn-Ser-Cyclohexylalanyl-Arg-$NH_2$

Folgende Verbindungen (Beispiele 14-22) werden wie in Referenzbeispiel 3 beschrieben, hergestellt.

14. H-Cys-Phe-D-Ala-Gly-Arg-Ile-Glu-Arg-Ile-Gly-Ala-Thr-

Ser-Gly-Leu-Gly-Cys-Pro-Ser-Phe-NH-$(CH_2)_4$-$NH_2$

15. H-Aoc-Ser-Cys-Phe-Gly-Gly-Arg-Ile-Asp-Arg-Ile-Gly-Ala-Gln-Ser-Gly-Leu-Gly-Cys-Asn-Ser-Phe-NH-$(CH_2)_8$-$NH_2$

16. H-Cys-Phe-Gly-Gly-Arg-Ile-Asp-Arg-Ile-Gly-Ala-Gln-Ser-Gly-Leu-Gly-Cys-Asn-Ser-Phe-NH-$(CH_2)_8$-$NH_2$

17. H-Cys-Phe-D-Ala-Gly-Arg-Ile-Asp-Arg-Ile-Gly-Ala-Gln-Ser-Gly-Leu-Gly-Cys-Asn-Ser-Phe-NH-$(CH_2)_6$-$NH_2$

18. H-Aoc-Cys-Phe-D-Ala-Gly-Arg-Ile-Glu-Arg-Ile-Gly-Ala-

Thr-Ser-Gly-Leu-Gly-Cys-Gly-Ser-Phe-NH-$(CH_2)_6$-$NH_2$

19. H-Arg-Cys-Phe-D-Ala-Gly-Arg-Ile-Asp-Arg-Ile-Gly-Ala-Gln-Ser-Gly-Leu-Gly-Cys-Asn-Ser-Phe-NH-$(CH_2)_6$-$NH_2$

20. H-Arg-Arg-Cys-Phe-D-Ala-Gly-Arg-Ile-Asp-Arg-Ile-Gly-Ala-Gln-Ser-Gly-Leu-Gly-Cys-Asn-Ser-Phe-NH-$(CH_2)_6$-$NH_2$

21. H-Aoc-Cys-Phe-D-Ala-Gly-Arg-Ile-Glu-Arg-Ile-Gly-Ala-Gln-Ser-Gly-Leu-Gly-Cys-Gly-D-Ala-Phe-NH-$(CH_2)_6$-$NH_2$

22. H-Aoc-Cys-Phe-D-Ala-Gly-Arg-Ile-Glu-Arg-Ile-Ala-Ala-

Thr-Ser-Gly-Leu-Gly-Cys-Pro-Ser-Phe-NH-$(CH_2)_4$-$NH_2$

Die Charakterisierung der Peptide erfolgt durch Aminosäureanalyse. Die Peptide wurden 12 Stunden bei 120 °C mit 6 N Salzsäure hydrolysiert. Cystein fiel dabei als Gemisch aus Cystein und Cysteinsäure an und war in allen Peptiden enthalten.

| Beispiel | 1 | 2 | 3 | 4 | 5 | 6 | 7 | 8 | 9 | 10 |
|---|---|---|---|---|---|---|---|---|---|---|
| ASP | 2.00 | 1.97 | 1.98 | 2.00 | | 1.98 | 1.95 | 1.98 | 1.97 | 2.09 |
| THR | | | | | 0.88 | | | | | |
| SER | 3.46 | 3.46 | 3.52 | 3.67 | 3.24 | 1.62 | 3.51 | 1.83 | 1.95 | 1.70 |
| GLU | 1.08 | 1.05 | 1.07 | 1.05 | 1.07 | 1.00 | 1.05 | 1.08 | 1.06 | 1.14 |
| PRO | | | | 1.11 | 1.05 | | | | | |
| GLY | 5.00 | 5.03 | 5.11 | 5.11 | 5.00 | 3.98 | 3.01 | 4.07 | 4.01 | 3.98 |
| ALA | 1.01 | 1.06 | 1.16 | 1.09 | 1.02 | 2.06 | 1.04 | 1.98 | 2.17 | 2.02 |
| ILE | 1.85 | 1.81 | 1.93 | 0.94 | 1.92 | 0.99 | 1.89 | 1.75 | 1.78 | 2.09 |
| LEU | 0.95 | 0.94 | 1.03 | 1.00 | 1.02 | 0.97 | 1.05 | 1.01 | 1.03 | 1.10 |
| TYR | 1.62 | 1.37 | 0.57 | 0.78 | 0.92 | 0.71 | 0.82 | | | |
| PHE | 1.04 | 1.07 | 1.99 | 1.99 | 2.16 | 1.98 | 1.95 | 2.04 | 2.00 | 2.08 |
| LYS | | | | | | | | 1.05 | 1.04 | |
| ARG | 2.88 | 2.88 | 2.91 | 2.97 | 3.06 | 2.94 | 2.97 | 2.04 | 1.97 | 3.00 |

| Beispiel | 11 | 12 | 13 | 14 | 15 | 16 | 17 | 18 | 19 | 20 | 21 | 22 |
|---|---|---|---|---|---|---|---|---|---|---|---|---|
| ASP | 2.05 | 2.11 | 2.00 | | 1.93 | 1.95 | 1.97 | | 2.00 | 1.98 | | |
| THR | | | | 0.92 | | | | 0.88 | | | | 1.06 |
| SER | 1.69 | 1.82 | 1.83 | 1.83 | 2.39 | 1.68 | 1.86 | 1.83 | 1.75 | 1.68 | 1.03 | 2.29 |
| GLU | 1.18 | 1.28 | 1.16 | 1.00 | 1.10 | 1.05 | 1.03 | 1.04 | 1.07 | 1.06 | 2.05 | 1.00 |
| PRO | | | | 0.98 | | | | | | | | 1.13 |
| GLY | 3.89 | 4.00 | 4.83 | 4.05 | 5.08 | 5.00 | 4.03 | 5.01 | 4.08 | 4.05 | 4.90 | 2.98 |
| ALA | 2.36 | 2.41 | 2.17 | 2.11 | 1.05 | 1.09 | 1.98 | 1.98 | 1.96 | 2.01 | 2.84 | 2.85 |
| ILE | 0.95 | 0.87 | 1.83 | 1.88 | 1.79 | 1.88 | 1.83 | 1.97 | 1.91 | 1.97 | 1.87 | 1.93 |
| LEU | 0.92 | | 1.13 | 1.16 | 0.97 | 0.99 | 1.07 | 1.03 | 1.00 | 1.00 | 1.00 | 1.04 |
| TYR | | | | | | | | | | | | |
| PHE | 2.12 | 2.04 | 1.00 | 2.00 | 1.98 | 1.97 | 1.99 | 2.00 | 1.89 | 1.97 | 1.83 | 1.83 |
| LYS | | | | | | | | | | | | |
| ARG | 3.00 | 2.90 | 2.95 | 1.98 | 2.00 | 1.84 | 2.00 | 1.95 | 2.87 | 3.98 | 1.80 | 1.81 |

## Ansprüche

1. Peptid der Formel I,

$$X\text{-}\overset{3}{V}\text{-}A\text{-}A^1\text{-}A^2\text{-}B\text{-}C\text{-}D\text{-}B\text{-}C\text{-}Gly\text{-}Ala\text{-}E\text{-}F\text{-}G\text{-}Leu\text{-}Gly\text{-}\overset{19}{Cys}\text{-}Z \quad (I)$$

in welcher

X Wasserstoff, $(C_1$-$C_5)$-Alkoxycarbonyl, $(C_6$-$C_{12})$-Aryloxycarbonyl, $(C_7$-$C_{13})$-Aralkyloxycarbonyl, $(C_1$-$C_6)$-Alkanoyl, $(C_7$-$C_{13})$-Aroyl, Arginyl, Lysyl, $\epsilon$-Aminocaproyl, Arginyl-Arginyl, Arginyl-Lysyl, Lysyl-Arginyl oder Lysyl-Lysyl bedeutet, gegebenenfalls in $\alpha$-Stellung verzweigtes und gegebenenfalls in $\omega$-Stellung durch Amino oder Guanidino monosubstituiertes $(C_1$-$C_{12})$-Alkylcarbonyl oder $(C_3$-$C_8)$-Cycloalkylcarbonyl bedeutet oder für Ser, Thr, Ser(Y), Thr(Y), Q oder Leu, jeweils in ihrer L-bzw. D-Konfiguration oder Ser-Ser, Thr-Thr, Ser-Thr, Q-Ser, Q-Thr, Thr-Q, Ser-Q, Ser(Y)-Ser oder Ser-Ser(Y) steht, wobei jede Aminosäure in ihrer L-bzw. D-Konfiguration vorliegen kann und die N-terminale Aminogruppe der Aminosäure oder des Dipeptid-Restes frei vorliegt (N-terminaler Rest = H) oder durch $(C_1$-$C_5)$-Alkoxycarbonyl, $(C_6$-$C_{12})$-Aryloxycarbonyl, $(C_7$-$C_{13})$-Aralkyloxycarbonyl, $(C_1$-$C_5)$-Alkanoyl, $(C_7$-$C_{13})$-Aroyl, Arginyl, Lysyl, $\epsilon$-Aminocaproyl, Arginyl-Arginyl, Arginyl-Lysyl, Lysyl-Arginyl oder Lysyl-Lysyl acyliert ist oder X fehlen kann;

V Cys oder, falls X fehlt, $\omega$-Thio$(C_2$-$C_8)$alkylcarbonyl, wie beispielsweise Mercaptopropionsäure, Mercaptoessigsäure, Mercaptobuttersäure, bedeutet;

A Phe, Tyr(Me), Tyr($Bu^t$), 4-Halogenphenylalanin, Trp oder einen L-2-Thienylalanin-Rest bedeutet;

$A^1$ Gly, Ala oder D-Ala bedeutet;

$A^2$ Gly, Ala oder D-Ala bedeutet; oder

$A^1$ und $A^2$ zusammen eine gegebenenfalls verzweigte $\omega$-Amino$(C_3$-$C_8)$-alkylcarbonsäure bedeutet;

B Arg, Lys, Orn oder einen L-Homoarginin-Rest bedeutet;

C Ile, Met, Phe, Trp, Leu, Ser, Thr, Val, His, Pro, Asn, Ser($Bu^t$), Cyclohexylalanin, tert. Butylglycin, Neopentylglycin oder einen L-2-Thienylalanin-Rest bedeutet;

D Asp, Glu, Gln, Asn, Phe, Leu, Ile, Trp, Pro, Tyr, Ala, Asp($OBu^t$), Asp(OBzl), Glu($OBu^t$), Glu(OBzl), einen 2-Thienylalanin-Rest, Aad, Tyr($bu^t$) oder Tyr(Me) bedeutet, wobei die Aminosäuren jeweils in ihrer L- oder D-Konfiguration vorliegen können;

E Gln, Thr oder Pro bedeutet;

F Ser, Thr, Pro, Ala, Ser($Bu^t$) oder Thr($Bu^t$), jeweils in ihrer L- oder D-Konfiguration bedeutet;

G Gly, Ala oder D-Ala bedeutet;

Q einen Rest der Formel IV,

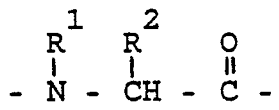 (IV)

worin
$R^1$ und $R^2$ zusammen mit den diese Reste tragenden Atomen ein heterocyclisches mono-, bi- oder tricyclisches Ringsystem mit 3 bis 15 C-Atomen bilden,
bedeutet;
Y tert.-Butyl oder einen gegebenenfalls geschützten oder teilgeschützten Glycosylrest bedeutet;
und
Z für einen Rest der Formel II steht,
-H-I-J-K-L-M (II)
worin H Asn, Ser, Q oder Thr, jeweils in ihrer L-oder D-Form, oder Gly bedeutet,
I Ser, Thr, Ala, Ser($Bu^t$), Thr($Bu^t$) oder Pro, jeweils in ihrer L- oder D-Form bedeuten;
J Phe, Trp, D-Phe, D-Trp, Tyr(Me), Cyclohexylalanyl, oder einen 2-Thienylalanin-Rest bedeutet;
K Arg, Lys, Orn oder eine Bindung bedeutet;
L Q, Gly, Tyr, Tyr($Bu^t$), Tyr(Me) oder eine Bindung bedeutet;
M Arg-OH, Arg-$NH_2$, OH, OR, $NH_2$, NHR′, Gly-Lys-Arg-OH, Gly-Lys-Arg-$NH_2$ oder L-Argininol bedeutet;
Q wie oben definiert ist;
R unverzweigtes ($C_1$-$C_6$)-Alkyl bedeutet und
R′ -$[CH_2]_n$-$NH_2$ oder -$[CH_2]_n$-NH-C(NH)$NH_2$,
wobei n ganzzahlig ist und für 3-8 steht, bedeutet; sowie dessen physiologisch verträgliche Salze, wobei die der Sequenz des natürlichen ANF entsprechenden Peptide der Formel III

(III) X-Cys-Phe-Gly-Gly-Arg-C-Asp-Arg-Ile-Gly-Ala-Gln-

-Ser-Gly-Leu-Gly-Cys-Z

worin X Ser, Ser-Ser, Arg-Ser-Ser oder Arg-Arg-Ser-Ser
C Ile oder Met,
Z Asn-Ser-Phe-K-L-M,
K Arg oder eine Bindung,
L Tyr oder eine Bindung und
M OH oder $NH_2$ bedeuten sowie deren Salze,
die in EP-A 231 752 (ZA 87/O 272; HOE 86/F 099J) beschriebenen Verbindungen;
rat-[$Mac^{105}$]ANF (101-126), rat-[$Mpr^{105}$]ANF(101-126), rat-[$Mbu^{105}$]ANF (101-126) und rat-[$Mpr^{105}$, D-$Ala^{107}$] ANF(105-126)
ausgenommen sind.

2. Peptid der Formel I gemäß Anspruch 1, in welcher Q Pro bedeutet, sowie dessen physiologisch verträgliche Salze.

3. Peptid der Formel I gemäß Anspruch 1 oder 2, in welcher Y tert.-Butyl bedeutet, sowie dessen physiologisch verträgliche Salze.

4. Peptid der Formel I gemäß einem der Ansprüche 1 bis 3, in welcher
X Wasserstoff, ($C_1$-$C_5$)-Alkoxycarbonyl, ($C_6$-$C_{12}$)-Aryloxycarbonyl, ($C_7$-$C_{13}$)-Aralkyloxycarbonyl, ($C_1$-$C_6$)-Alkanoyl, ($C_7$-$C_{13}$)-Aroyl, Arginyl, Lysyl oder Arginyl-Arginyl bedeutet,
gegebenenfalls in α-Stellung verzweigtes und gegebenenfalls in ω-Stellung durch Amino oder Guanidino monosubstituiertes ($C_1$-$C_{12}$)-Alkylcarbonyl oder ($C_3$-$C_8$)-Cycloalkylcarbonyl bedeuten oder für Ser, Thr oder Q, jeweils in ihrer L- bzw. D-Konfiguration oder Ser-Ser, Thr-Thr, Ser-Thr, Pro-Ser, Pro-Thr, Thr-Pro oder Ser-Pro, vorzugsweise Ser-Ser steht, wobei jede Aminosäure in ihrer L- bzw. D-Konfiguration vorliegen kann und die N-terminale Aminogruppe der Aminosäure oder des Dipeptid-Restes frei vorliegt oder durch ($C_1$-$C_5$)-Alkoxycarbonyl, ($C_6$-$C_{12}$)-Aryloxycarbonyl, ($C_7$-$C_{13}$)-Aralkyloxycarbonyl, ($C_1$-$C_6$)-Alkanoyl, ($C_7$-$C_{13}$)-Aroyl, Arginyl, Lysyl oder Arginyl-Arginyl acyliert ist oder X fehlen kann;
V Cys oder, falls X fehlt, Mercaptopropionsäure, Mercaptoessigsäure oder Mercaptobuttersäure bedeutet;
A Phe, Tyr(Me), Tyr($Bu^t$), 4-Halogenphenylalanin oder einen L-2-Thienylalanin-Rest, vorzugsweise Phe

bedeutet;

A' Gly, Ala oder D-Ala bedeutet;

$A^2$ Gly, Ala oder D-Ala bedeutet; oder

$A^1$ und $A^2$ zusammen eine gegebenenfalls verzweigte ω-Amino-($C_3$-$C_8$)-alkylcarbonsäure bedeutet;

B Arg oder Lys bedeutet;

C Ile, Cyclohexylalanin, tert.-Butylglycin, Neopentylglycin, Phe, Leu, Val oder einen L-2-Thienylalanin-Rest bedeutet;

D Asp, Glu, Gln, Asn, Leu, Ile, Trp, Asp($OBu^t$), Glu($OB^t$), Glu(OBzl) oder einen 2-Thienylalanin-Rest, vorzugsweise Asp, Glu oder Leu bedeutet, wobei die Aminosäuren jeweils in ihrer L- oder D-Konfiguration vorliegen können;

E Gln, Thr oder Pro bedeutet;

F Ser, Thr oder Ala, vorzugsweise Ser oder Ala, jeweils in ihrer L- oder D-Konfiguration bedeutet;

G Gly, Ala oder D-Ala bedeutet und

Z für ein Rest der Formel II steht,

worin H Asn, Ser, Pro oder Thr, vorzugsweise Pro, Thr oder Asn, jeweils in ihrer L- oder D-Form, oder Gly bedeutet;

I Ser, Thr, Ala oder Ser($Bu^t$), vorzugsweise Ser, jeweils in ihrer L- oder D-Form bedeutet;

J Phe, Tyr(Me), Cyclohexylalanyl oder einen L-2-Thienylalanin-Rest, vorzugsweise Phe bedeutet;

K Arg, Lys, Orn oder eine Bindung bedeutet;

L Tyr, Q, Gly, Tyr(Me) oder eine Bindung bedeutet;

M Arg-OH, Arg-$NH_2$, OH, OR, $NH_2$, NHR', Gly-Lys-Arg-OH oder Gly-Lys-Arg-$NH_2$ bedeutet;

R unverzweigtes ($C_1$-$C_6$)-Alkyl bedeutet

und

R' -$[CH_2]_n$-$NH_2$ oder -$[CH_2]_n$-NH-C(NH)$NH_2$, wobei n ganzzahlig ist und für 3-8 steht, bedeutet sowie dessen physiologisch verträgliche Salze, wobei Peptide der Formel III, die in EP-A 231 752 beschriebenen Verbindungen sowie deren Salze, rat-[Mac[105]]ANF(101-126), rat-[Mpr[105]]ANF(101-126), rat-[Mbu[105]]ANF (101-126) und rat-[Mpr[105], D-Ala[107]]ANF (105-126) ausgenommen sind.

5. Peptid der Formel I, gemäß einem der Ansprüche 1 bis 4, in welcher

X Wasserstoff, Arginyl oder Arginyl-Arginyl, Ser, Q oder Ser-Ser bedeutet, wobei Q und Ser jeweils in der L- oder D-Konfiguration vorliegen können und die N-terminale Aminogruppe des Aminosäure- oder Dipeptid-Restes frei vorliegt oder durch Arginyl oder Arginyl-Arginyl acyliert ist oder X fehlen kann;

V Cys oder, falls X fehlt, Mercaptopropionsäure bedeutet;

A Phe bedeutet;

B Arg oder Lys bedeutet;

C Ile, Leu, Val oder einen L-2-Thienylalanin-Rest bedeutet;

D Asp, Gln, Leu, Ile, As-($OBu^t$), Glu($OBu^t$), Tyr($Bu^t$) oder Tyr(Me) bedeutet;

E Gln, Thr oder Pro bedeutet;

F Ser oder Ala jeweils in ihrer L- oder D- Konfiguration bedeutet;

G Gly, Ala, oder D-Ala bedeutet

und

Z für einen Rest der Formel II steht

worin H Asn, Pro oder Thr, jeweils in ihrer L- oder D-Form, oder Gly bedeutet;

I Ser, Thr oder Ala jeweils in ihrer L- oder D-Form bedeutet;

J Phe, Tyr(Me), Cyclohexylalanyl oder einen 2-Thienylalanin-Rest bedeutet;

K Arg, Lys oder eine Bindung bedeutet;

L Tyr, Tyr($Bu^t$) oder eine bindung bedeutet;

M OH, $NH_2$, NHR', Gly-Lys-Arg-OH oder Gly-Lys-Arg-$NH_2$ bedeutet und

R' -$[CH_2]_2$-$NH_2$ oder -$[CH_2]_n$-NH-C(NH)$NH_2$ bedeutet,

wobei n ganzzahlig ist und für 3-8 steht; sowie deren physiologisch verträgliche Salze, wobei Peptide der Formel III, die in EP-A 231 752 beschriebenen Verbindungen sowie deren Salze, rat-[Mac[105]]ANF(101-126), rat-[Mpr[105]]ANF (101-126), rat-[Mbu[105], D-Ala[107]]ANF(101-126) und rat-[Mpr[105]]ANF(105-126) ausgenommen sind.

6. Verfahren zur Herstellung eines Peptides der Formel I, gemäß einem der Ansprüche 1 bis 5, dadurch gekennzeichnet ist, daß man

a) ein Fragment mit C-terminaler freier Carboxylgruppe oder dessen aktiviertes Derivat mit einem entsprechenden Fragment mit N-terminaler freier Aminogruppe umsetzt oder

b) das Peptid stufenweise aufbaut, in der nach (a) oder (b) erhaltenen Verbindung gegebenenfalls eine oder mehrere zum Schutz anderer Funktionen temporär eingeführte Schutzgruppen abspaltet, eine Disulfidbrücke zwischen den beiden Cys-Resten knüpft, wobei die letztgenannten beiden Maßnahmen auch in umgekehrter Reihenfolge durchgeführt werden können, und die so erhaltenen Verbindung der Formel I gegebenenfalls in ihr physiologisch verträgliches Salz überführt.

7. Verfahren gemäß Ansprüch 6, dadurch gekennzeichnet, daß ein Fragment während des Syntheseablaufs an einem Festkörper kovalent gebunden ist.

8. Verwendung einer Verbindung gemäß einem der Ansprüche 1 bis 5 als Heilmittel.

9. Verwendung einer Verbindung gemäß einem der Ansprüche 1 bis 5 als Diuretikum.

10. Verwendung einer Verbindung gemäß einem der Ansprüche 1 bis 5 als vasorelaxierendes Mittel.

11. Verwendung einer Verbindung gemäß einem der Ansprüche 1 bis 5 oder einer Verbindung der Formel III, in welcher X, C, Z, K, L und M wie im Anspruch 1 definiert sind bzw. deren physiologisch verträglichen Salze als immunmodulierendes Mittel.

12. Pharmazeutische Zubereitung enthaltend eine Verbindung gemäß einem der Ansprüche 1 bis 5.

13. Verwendung einer Verbindung gemäß einem der Ansprüche 1 bis 5 als Mittel bei der Behandlung des Glaukoms und/oder der Herabsetzung des Augeninnendrucks.

14. Verwendung einer Verbindung gemäß einem der Ansprüche 1 bis 5 bei der Behandlung der Niereninsuffizienz.

Patentansprüche für die folgenden Vertragsstaaten: ES, GR

1. Verfahren zur Herstellung eines Peptides der Formel I

$$X\text{-}\overset{3}{V}\text{-}A\text{-}A^1\text{-}A^2\text{-}B\text{-}C\text{-}D\text{-}B\text{-}C\text{-}Gly\text{-}Ala\text{-}E\text{-}F\text{-}G\text{-}Leu\text{-}Gly\text{-}\overset{19}{Cys}\text{-}Z \qquad (I)$$

in welcher

X Wasserstoff, $(C_1\text{-}C_5)$-Alkoxycarbonyl, $(C_6\text{-}C_{12})$-Aryloxycarbonyl, $(C_7\text{-}C_{13})$-Aralkyloxycarbonyl, $(C_1\text{-}C_6)$-Alkanoyl, $(C_7\text{-}C_{13})$-Aroyl, Arginyl, Lysyl, $\epsilon$-Aminocaproyl, Arginyl-Arginyl, Arginyl-Lysyl, Lysyl-Arginyl oder Lysyl-Lysyl bedeutet, gegebenenfalls in $\alpha$-Stellung verzweigtes und gegebenenfalls in $\omega$-Stellung durch Amino oder Guanidino monosubstituiertes $(C_1\text{-}C_{12})$-Alkylcarbonyl oder $(C_3\text{-}C_8)$-Cycloalkylcarbonyl bedeutet oder für Ser, Thr, Ser(Y), Thr(Y), Q oder Leu, jeweils in ihrer L-bzw. D-Konfiguration oder Ser-Ser, Thr-Thr, Ser-Thr, Q-Ser, Q-Thr, Thr-Q, Ser-Q, Ser(Y)-Ser oder Ser-Ser(Y) steht, wobei jede Aminosäure in ihrer L- bzw. D-Konfiguration vorliegen kann und die N-terminale Aminogruppe der Aminosäure oder des Dipeptid-Restes frei vorliegt (N-terminaler Rest = H) oder durch $(C_1\text{-}C_5)$-Alkoxycarbonyl, $(C_6\text{-}C_{12})$-Aryloxycarbonyl, $(C_7\text{-}C_{13})$-Aralkyloxycarbonyl, $(C_1\text{-}C_6)$-Alkanoyl, $(C_7\text{-}C_{13})$-Aroyl, Arginyl, Lysyl, $\epsilon$-Aminocaproyl, Arginyl-Arginyl, Arginyl-Lysyl, Lysyl-Arginyl oder Lysyl-Lysyl acyliert ist oder X fehlen kann;

V Cys oder, falls X fehlt, w-Thio$(C_2\text{-}C_8)$alkylcarbonyl, wie beispielsweise Mercaptopropionsäure, Mercaptoessigsäure, Mercaptobuttersäure, bedeutet;

A Phe, Tyr(Me), Tyr($Bu^t$), 4-Halogenphenylalanin, Trp oder einen L-2-Thienylalanin-Rest bedeutet;

$A^1$ Gly, Ala oder D-Ala bedeutet;

$A^2$ Gly, Ala oder D-Ala bedeutet; oder

$A^1$ und $A^2$ zusammen eine gegebenenfalls verzweigte $\omega$-Amino$(C_3\text{-}C_8)$alkylcarbonsäure bedeutet;

B Arg, Lys, Orn oder einen L-Homoarginin-Rest bedeutet;

C Ile, Met, Phe, Trp, Leu, Ser, Thr, Val, His, Pro, Asn, Ser($Bu^t$), Cyclohexylalanin, tert. Butylglycin, Neopentylglycin oder einen L-2-Thienylalanin-Rest bedeutet;

D Asp, Glu, Gln, Asn, Phe, Leu, Ile, Trp, Pro, Tyr, Ala, Asp($OBu^t$), Asp(OBzl), Glu($OBu^t$), Glu(OBzl), einen 2-Thienylalanin-Rest, Aad, Tyr($Bu^t$) ode Tyr(Me) bedeutet, wobei die Aminosäuren jeweils in ihrer L- oder D-Konfiguration vorliegen können;

E Gln, Thr oder Pro bedeutet;

F Ser, Thr, Pro, Ala, Ser($Bu^t$) oder Thr($Bu^t$), jeweils in ihrer L- oder D-Konfiguration bedeutet;

G Gly, Ala oder D-Ala bedeutet;

Q einen Rest der Formel IV,

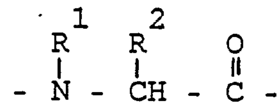 (IV)

worin

$R^1$ und $R^2$ zusammen mit den diese Reste trangenden Atomen ein heterocyclisches mono-, bi- oder tricyclisches Ringsystem mit 3 bis 15 C-Atomen bilden,

bedeutet;

Y tert.-Butyl oder einen gegebenenfalls geschützten oder teilgeschützten Glycosylrest bedeutet;

und

Z für einen Rest der Formel II steht,

-H-I-J-K-L-M (II)

worin H Asn, Ser, Q oder Thr, jeweils in ihrer L-oder D-Form, oder Gly bedeutet,

I Ser, Thr, Ala, Ser($Bu^t$), Thr($Bu^t$) oder Pro, jeweils in ihrer L- oder D-Form bedeuten;

J Phe, Trp, D-Phe, D-Trp, Tyr(Me), Cyclohexylalanyl, oder einen 2-Thienylalanin-Rest bedeutet;

K Arg, Lys, Orn oder eine Bindung bedeutet;

L Q, Gly, Tyr, Tyr($Bu^t$), Tyr(Me) oder eine Bindung bedeutet;

M Arg-OH, Arg-$NH_2$, OH, OR, $NH_2$, NHR′, Gly-Lys-Arg-OH, Gly-Lys-Arg-$NH_2$ oder L-Argininol bedeutet;

Q wie oben definiert ist;

R unverzweigtes ($C_1$-$C_6$)-Alkyl bedeutet und

R′ -$[CH_2]_n$-$NH_2$ oder -$[CH_2]_n$-NH-C(NH)$NH_2$,

wobei n ganzzahlig ist und für 3-8 steht, bedeutet; sowie dessen physiologisch verträgliche Salze, wobei die der Sequenz des natürlichen ANF entsprechenden Peptide der Formel III

(III) X-Cys-Phe-Gly-Gly-Arg-C-Asp-Arg-Ile-Gly-Ala-Gln-

-Ser-Gly-Leu-Gly-Cys-Z

worin X Ser, Ser-Ser, Arg-Ser-Ser oder Arg-Arg-Ser-Ser

C Ile oder Met,

Z Asn-Ser-Phe-K-L-M,

K Arg oder eine Bindung,

L Tyr oder eine Bindung und

M OH oder $NH_2$ bedeuten sowie deren Salze,

die in EP-A 231 752 (ZA 87/O 272; HOE 86/F 099J) beschriebenen Verbindungen:

rat-[$Mac^{105}$]ANF(101-126), rat-[$Mpr^{105}$]ANF(101-126), rat-[$Mbu^{105}$]ANF (101-126) und rat-[$Mpr^{105}$, D-$Ala^{107}$] ANF(105-126)

ausgenommen sind,

dadurch gekennzeichnet, daß man

a) ein Fragment mit C-terminaler freier Carboxylgruppe oder dessen aktiviertes Derivat mit einem entsprechenden Fragment mit N-terminaler freier Aminogruppe umsetzt oder

b) das Peptid stufenweise aufbaut, in der nach (a) oder (b) erhaltenen Verbindung gegebenenfalls eine oder mehrere zum Schutz anderer Funktionen temporär eingeführte Schutzgruppen abspaltet, eine Disulfidbrücke zwischen den beiden Cys-Resten knüpft, wobei die letztgenannten beiden Maßnahmen auch in umgekehrter Reihenfolge durchgeführt werden können, und die so erhaltenen Verbindung der Formel I gegebenenfalls in ihr physiologisch verträgliches Salz überführt.

2. Verfahren gemäß Anspruch 1, dadurch gekennzeichnet, daß man ein Peptid der Formel I herstellt, in welcher Q Pro bedeutet, sowie dessen physiologisch verträgliche Salze.

3. Verfahren gemäß Anspruch 1 oder 2, dadurch gekennzeichnet, daß man ein Peptid der Formel I herstellt, in welcher Y tert.-Butyl bedeutet, sowie dessen physiologisch verträgliche Salze.

4. Verfahren gemäß einem der Ansprüche 1 bis 3, dadurch gekennzeichnet, daß man ein Peptid der Formel I herstellt, in welcher

X Wasserstoff, ($C_1$-$C_5$)-Alkoxycarbonyl, ($C_6$-$C_{12}$)-Aryloxycarbonyl, ($C_7$-$C_{13}$)-Aralkyloxycarbonyl, ($C_1$-$C_6$)-Alkanoyl, ($C_7$-$C_{13}$)-Aroyl, Arginyl, Lysyl oder Arginyl-Arginyl bedeutet,

gegebenenfalls in α-Stellung verzweigtes und gegebenenfalls in ω-Stellung durch Amino oder Guanidino

monosubstituiertes ($C_1$-$C_{12}$)-Alkylcarbonyl oder ($C_3$-$C_8$)-Cycloalkylcarbonyl bedeuten oder für Ser, Thr oder Q, jeweils in ihrer L- bzw. D-Konfiguration oder Ser-Ser, Thr-Thr, Ser-Thr, Pro-Ser, Pro-Thr, Thr-Pro oder Ser-Pro, vorzugsweise Ser-Ser steht, wobei jede Aminosäure in ihrer L- bzw. D-Konfiguration vorliegen kann und die N-terminale Aminogruppe der Aminosäure oder den Dipeptid-Restes frei vorliegt oder durch ($C_1$-$C_5$)-Alkoxycarbonyl, ($C_6$-$C_{12}$)-Aryloxycarbonyl, ($C_7$-$C_{13}$)-Aralkyloxycarbonyl, ($C_1$-$C_6$)-Alkanoyl, ($C_7$-$C_{13}$)-Aroyl, Arginyl, Lysyl oder Arginyl-Arginyl acyliert ist oder X fehlen kann;

V Cys oder, falls X fehlt, Mercaptopropionsäure, Mercaptoessigsäure oder Mercaptobuttersäure bedeutet;

A Phe, Tyr (Me), Tyr($Bu^t$), 4-Halogenphenylalanin oder einen L-2-Thienylalanin-Rest, vorzugsweise Phe bedeutet;

$A^1$ Gly, Ala oder D-Ala bedeutet;

$A^2$ Gly, Ala oder D-Ala bedeutet; oder

$A^1$ und $A^2$ zusammen eine gegebenenfalls verzweigte $\omega$-Amino-($C_3$-$C_8$)-alkylcarbonsäure bedeutet;

B Arg oder Lys bedeutet;

C Ile, Cyclohexylalanin, tert.-Butylglycin, Neopentylglycin, Phe, Leu, Val oder einen L-2-Thienylalanin-Rest bedeutet;

D Asp, Glu, Gln, Asn, Leu, Ile, Trp, Asp($OBu^t$), Glu($OBu^t$), Glu(OBzl) oder einen 2-Thienylalanin-Rest, vorzugsweise Asp, Glu oder Leu bedeutet, wobei die Aminosäuren jeweils in ihrer L- oder D-Konfiguration vorliegen können;

E Gln, Thr oder Pro bedeutet;

F Ser, Thr oder Ala, vorzugsweise Ser oder Ala, jeweils in ihrer L- cder D-Konfiguration bedeutet;

G Gly, Ala oder D-Ala bedeutet und

Z für ein Rest der Formel II steht,

worin H Asn, Ser, Pro oder Thr, vorzugsweise Pro, Thr oder Asn, jeweils in ihrer L- oder D-Form, oder Gly bedeutet;

I Ser, Thr, Ala oder Ser($Bu^t$), vorzugsweise Ser, jeweils in ihrer L- oder D-Form bedeutet; J Phe, Tyr(Me), Cyclohexylalanyl oder einen L-2-Thienylalanin-Rest, vorzugsweise Phe bedeutet;

K Arg, Lys, Orn oder eine Bindung bedeutet;

L Tyr, Q, Gly, Tyr(Me) oder eine Bindung bedeutet;

M Arg-OH, Arg-$NH_2$, OH, OR, $NH_2$, NHR′, Gly-Lys-Arg-OH oder Gly-Lys-Arg-$NH_2$ bedeutet;

R unverzweigtes ($C_1$-$C_6$)-Alkyl bedeutet

und

R′ -$[CH_2]_n$-$NH_2$ oder -$[CH_2]_n$-NH-C(NH)$NH_2$, wobei n ganzzahlig ist und für 3-8 steht, bedeutet sowie deren physiologisch verträgliche Salze, wobei Peptide der Formel III, die in EP-A 231 752 beschriebenen Verbindungen sowie deren Salze, rat-[$Mac^{105}$]ANF(101-126), rat-[$Mpr^{105}$]ANF(101-126), rat-[$Mbu^{105}$]ANF (101-126) und rat-[$Mpr^{105}$, D-$Ala^{107}$]ANF (105-126) ausgenommen sind.

5. Verfahren gemäß einem der Ansprüche 1 bis 4, dadurch gekennzeichnet, daß man ein Peptid der Formel I herstellt, in welcher

X Wasserstoff, Arginyl oder Arginyl-Arginyl, Ser, Q oder Ser-Ser bedeutet, wobei Q und Ser jeweils in der L- oder D-Konfiguration vorliegen können und die N-terminale Aminogruppe des Aminosäure- oder Dipeptid-Restes frei vorliegt oder durch Arginyl oder Arginyl-Arginyl acyliert ist oder X fehlen kann;

V Cys oder, falls X fehlt, Mercaptopropionsäure bedeutet;

A Phe bedeutet;

B Arg oder Lys bedeutet;

C Ile, Leu, Val oder einen L-2-Thienylalanin-Rest bedeutet;

D Asp, Gln, Leu, Ile, Asp($OBu^t$), Glu($OBu^t$), Tyr($Bu^t$) oder Tyr(Me) bedeutet;

E Gln, Thr oder Pro bedeutet;

F Ser oder Ala jeweils in ihrer L- oder D-Konfiguration bedeutet;

G Gly, Ala, oder D-Ala bedeutet und

Z für einen Rest der Formel II steht

worin H Asn, Pro oder Thr, jeweils in ihrer L- oder D-Form, oder Gly bedeutet;

I Ser, Thr oder Ala jeweils in ihrer L- oder D-Form bedeutet;

J Phe, Tyr(Me), Cyclohexylalanyl oder einen 2-Thienylalanin-Rest bedeutet;

K Arg, Lys oder eine Bindung bedeutet;

L Tyr, Tyr($Bu^t$) oder eine Bindung bedeutet;

M OH, $NH_2$, NHR′, Gly-Lys-Arg-OH oder Gly-Lys-Arg-$NH_2$ bedeutet und

R′ -$[CH_2]_2$-$NH_2$ oder -$[CH_2]_n$-NH-C(NH)$NH_2$ bedeutet, wobei n ganzzahlig ist und für 3-8 steht;

sowie deren physiologisch verträgliche Salze, wobei Peptide der Formel III, die in EP-A 231 752 beschriebenen Verbindungen sowie deren Salze, rat-[Mac$^{105}$]ANF(101-126), rat-[Mpr$^{105}$]ANF(101-126), rat-[Mbu$^{105}$]ANF(101-126) und rat-[Mpr$^{105}$, D-Ala$^{107}$]ANF(105-126) ausgenommen sind.

6. Verfahren zur Herstellung eines pharmazeutischen Mittels, enthaltend ein Peptid der Formel I gemäß einem der Ansprüche 1 bis 5, dadurch gekennzeichnet, daß man dieses und einen Träger in eine geeignete Darreichungsform bringt.